# EUROPEAN PATENT APPLICATION

(11) **EP 3 375 410 A1**
(43) Date of publication of application: **19.09.2018**
(21) Application number: 17160686.6
(22) Date of filing: 13.03.2017
(51) Int. Cl.: A61F 2/16

(54) **ACCOMMODATIVE LENS DEVICE**

(71) Applicant: KEJAKO SA, 1228 Plan-les-Ouates (CH)
(72) Inventor: MAURER, Aurélien, 1228 Plan-les-Ouates (CH); ENFRUN, David, 1228 Plan-les-Ouates (CH); BOS, Gilles Claude, 1228 Plan-les-Ouates (CH)
(74) Representative: Petit, Maxime

(57) **Abstract**

The invention concerns an implant that comprises an anterior part and a posterior part extending along a longitudinal axis and having respectively an anterior (A) and a posterior pole (E) both located on axis. The anterior and posterior parts extend each radially relative to axis, on either side thereof, the anterior and posterior parts having each two portions located on both sides of axis respectively when viewed in a sagittal plane. Each portion of the anterior part has a radial extension that increases from anterior pole (A) to a point (B, B') where the anterior part ends and the posterior part begins, each portion of the posterior part having a radial extension decreasing from point (B, B') to the posterior pole (E). The outer outline of each portion of the anterior part forms a curve having a radius of curvature that is greater at anterior pole (A) than at point (B, B'). The implant is made of one or more materials that have elastic or visco-elastic and cohesive properties in a solid state such that the shear modulus is between 10 Pa and 10 kPa.

## Description

The invention concerns an accommodative lens device which may provide lens physical restoration, and more particularly, an intra cortical lens implant and an intra capsular lens implant.

Presbyopia most often presents as a near vision deficiency, the inability to read small print, especially in dim lighting after about 40-45 years of age.

Presbyopia, or the loss of accommodative amplitude with age, relates to the eye's inability to change the shape of the natural crystalline lens, which allows a person to change focus between far and near, and occurs in essentially 100% of the population. Accommodative amplitude has been shown to decline with age steadily through the fifth decade of life.

Historically, studies have generally attributed loss of accommodation to the hardening of the crystalline tens with age and, more specifically, to an increase in the Young's Modulus of Elasticity of the lens material.

According to the Helmholtz theory, presbyopia is caused by increased stiffness of the lens with age which makes it harder to change the shape of the lens during accommodation and thus to focus the incoming light properly on to the retina.

More recent studies have examined the effect of aging on the relative change in material properties between the nucleus and the cortex. These studies have provided various theories and data with respect to the hardening of the lens. In a general manner, such studies have essentially proposed the theory according to which the loss of flexibility is the result of an increase in the Young's Modulus of Elasticity of the nucleus and/or cortex material. Such studies have viewed this hardening as the primary factor in the loss of accommodative amplitude with age and hence the cause of presbyopia.

Due to the phases of growth, the eye has various nuclei which differ in age and are superimposed on each other in an onion-like manner. The innermost and thus oldest nucleus hardens as it ages.

As the eye ages, there are also age related changes to these structures that include the development of intermolecular bonding, mostly disulfide bonding, the compaction of tissue, the breakdown of some of the original attachments, and the yellowing or darkening of older lens areas.

Actually, compaction of the lens occurs with aging. The number of lens fibers that grow every year is relatively constant throughout life. However, the size of the lens does not become as large as expected from new fiber growth. The lens grows from birth through age 3, from 6 mm to 7.2 mm or 20% growth in only three years. Then during the next approximate decade, growth is from 7.2 mm to 9 mm or 25%. However, this is over a three times longer period of 9 years. Over the next approximate two decades, from age 12 to age 36 the lens grows from 9 mm to 9.6 or 6.7% growth in 24 years, showing a dramatically slowing observed growth rate, while it is believed there is a relatively constant rate of fiber growth during this period. Finally, in the last approximately two decades described, from age 36 to age 54, the lens grows by a tiny fraction of its youthful growth, from 9.6 to 9.8 mm or 2.1% in 18 years. Although there is a geometry effect of needing more lens fibers to fill larger outer shells, the size of the older lens is considerably smaller than predicted by fiber growth rate models, which consider geometry effects. Fiber compaction including nuclear fiber compaction is thought to explain these observations.

Presbyopia may be corrected by the use of external optical correction devices including reading glasses, progressive spectacles, bifocal or trifocal lenses, contact lenses etc. However, many patients would like to avoid these external optical correction devices, especially the use of reading glasses considered as very troublesome.

All other commercially available products which may help restore accommodation are overall surgical and invasive.

A single method has been approved by the FDA as a temporary cure for presbyopia: conductive keratoplasty, e.g. as described in US 2007/038210. However, the effect only lasts for 3-6 months.

Another method, called lentotomy e.g. as described in US 2014/378955 and US 2010/191230 is based on cutting gliding planes inside the intact lens to allow for greater flexibility of the lens by the use of a femtosecond laser. This method is not commercially available.

None of the above methods has been satisfactory yet.

There still exists a need for an accommodative lens device, in particular an implant, which restores flexibility, and therefore increase visual accommodative amplitude, in a human eye.

There also exists a more general need for a new accommodative lens device which may remedy other optical disorders while providing actual dynamic visual accommodation.

An embodiment of the invention is defined in claim 1.

This new intra cortical lens implant makes it possible to bring flexibility or elasticity in a patient's eye which has been affected by ophthalmic disorders such as presbyopia. Thus, the implanted eye is provided with a new accommodative amplitude which increases the natural accommodative amplitude of the aged non-implanted eye. The new intra cortical lens implant may provide the patient with an accommodative amplitude (or increased accommodative amplitude) that gives comfortable near and far sight in the day to day patient life (e.g. the patient is capable of far distance viewing and reading at a distance of about 30 cm). Given the patient age, he/she will be given back an amplitude of accommodation that can be found in a natural younger crystalline lens (several years backward). This will make it possible to compensate for the loss of visual accommodation that affects the aged non-implanted eye.

This intra cortical lens implant acts as a soft or elastic core in a surrounding more rigid/stiff natural crystalline lens and helps reduce the rigidity/stiffness of the natural lens after implantation. Thus it is possible to come back to initial overall relative mechanical properties (nucleus softer than cortex) that can be found on a younger crystalline lens. The rigidity/stiffness features the relationship between a mechanical stress that is applied to an object and the displacement or deformation of the latter induced by this stress.

The intra cortical lens implant may be made of one material or a combination or mixture of several materials in a solid state that confer mechanical flexibility or elasticity properties to the intra cortical lens implant. The one or more materials have elastic or viscoelastic properties (e.g. a gel has visco-elastic properties and may be in a solid state) that are featured by a shear modulus greater than 10 Pa and less than 10 kPa.

The one or more materials in a solid state have cohesive properties. This means that an intra cortical lens implant made of such material or materials is has its own shape when not implanted, i.e. its shape is maintained without specific external constraints.

The implant made of one or more materials with elastic or visco-elastic properties as defined above can therefore be elastically deformed. This means that the implant is able to deform in an elastic manner when subjected to constraints and therefore to change its initial shape. When the constraints do no longer exert on the implant, the latter may take over its initial shape. This deformation under constraint may be repeated a great number of times in the course of life of the implant.

The one or more solid materials have a refractive index that is suitable for being used in a crystalline lens. More particularly, the refractive index of the material(s) is such that when the intra cortical lens implant is put in place in the crystalline lens, the refractive index of the latter in its most flattened shape is adapted to the far vision.

The new intra cortical lens implant as defined in claim 1 may deal with other optical disorders linked with lens aging (either as a preventive or curative treatment to such disorders) when at least part of a natural crystalline lens is replaced by an artificial accommodative lens that is not subject to such disorders.

To be noted that the intra cortical lens implant may be put in place in one eye or two eyes of a patient when dealing with a presbyopia disorder or other optical disorders.

In the embodiment of claim 1 the anterior pole A and posterior pole E both located on the longitudinal axis of the implant (polar axis) are fixed points of the implant whatever the sagittal plane of the implant (plane including the longitudinal axis). In contrast, the points B and B' that are the most radially extended points of the implant relative to the longitudinal axis may take different positions relative to this axis and to the poles A and E in different sagittal planes. In other words, the axis passing by the points B and B' may be at different angles to the polar axis AE. The four points A, B, E and B' define in every sagittal plane a quadrilateral that may vary in shape from one plane to the other or remain the same for an axi-symmetrical implant. The outer shape of the implant may therefore extend based on such a quadrilateral.

According to possible features of the implant:
- the anterior part has a convex shape when viewed in a sagittal plane;
- the outer outline of each portion of the anterior part is a continuous curve with a radius of curvature R that overall decreases from the anterior pole A to point B, B' in a sagittal plane;
- each portion of the posterior part has an outer outline that forms a continuous curve between the point B, B' and the posterior pole E when viewed in a sagittal plane;
- the outer outline of each portion of the posterior part includes two points C, D, respectively C', D', located on the curve between the point B, respectively B', and the posterior pole E and that form two inflexion points for the curve;
- the two points C, D, respectively C', D', are located on a straight line between the point B, respectively B', and the posterior pole E;
- each curve between the point B, respectively B', and the posterior pole E includes two side portions B, C, respectively B', C', and D, E, respectively D', E, flanking a central portion C, D, respectively C', D', the two side portions having a curvature of the same type, convex or concave, and the central portion having a curvature of the opposite type, concave or convex;
- the intra cortical lens implant has an axi-symmetrical shape relative to its polar axis when viewed in a sagittal plane;
- the intra cortical lens implant has not an axi-symmetrical shape relative to its polar axis when viewed in a sagittal plane;
- the intra cortical lens implant has an axi-symmetrical shape relative to its polar axis when viewed in a front plane that is perpendicular to the sagittal plane;
- the intra cortical lens implant has not an axi-symmetrical shape relative to its polar axis when viewed in a front plane that is perpendicular to the sagittal plane;

A second embodiment concerns an intra capsular lens implant intended to wholly fill in the capsular bag in an eye as defined in claim 12. The intra capsular lens implant comprises a core part and a shell part that surrounds the latter. The core part may act as the intra cortical lens implant of claim 1 and more particularly comprises the geometrical features of this intra cortical lens implant. The core part is surrounded by the shell part whereas the cortical lens implant of claim 1 is intended to be surrounded by natural tissues of the crystalline lens when put in place in the latter. Both the core part and the shell part may be made of one or more materials that have elastic or visco-elastic and cohesive properties in a solid state such that the shear modulus is greater than 10 Pa and less than 10 kPa. Alternatively, the shell part may be made of one or more of the previously described materials while the core part may be made of a fluid (e.g. a liquid or a gas). All that has been described above for the material(s) in relation with the implant of claim 1 also applies here.

When both the core part and the shell part are made of one or more of the above materials, the shell part may be made of one or more materials of which the shear modulus is greater than that of the core part.

Both the one or more materials and the fluid have a refractive index that is suitable for being used in a crystalline lens. More particularly, the refractive index is such that when the intra capsular lens implant is put in place in the crystalline lens, the refractive index of the latter in its most flattened shape is adapted to the far vision.

The whole intra capsular lens implant is to be inserted within a natural crystalline lens. The inner of the lens has been cleared beforehand so as to leave unaffected the capsular bag only and the intra capsular lens implant then may perfectly fill in the void created inside the capsular bag.

Alternatively, the intra capsular lens implant may comprise a hollow shell that is intended to be inserted within a natural crystalline lens (the inner of the lens has been cleared beforehand so as to leave unaffected the capsular bag only) and subsequently filled in by one or more visco-elastic materials or a fluid as defined above so as to act as the core part. In this alternative, the capsular bag together with the surrounding external shell part of the hollow shell play the role of a more rigid part of the lens while the core part is more flexible or elastic.

The new intra capsular lens implant as defined in claim 12 may deal with other optical disorders (either as a preventive or curative treatment to such disorders) when the whole natural crystalline lens is replaced by an artificial accommodative lens that is not subject to such disorders.

According to other possible features of the intra capsular lens implant:
- the shell part of the intra capsular lens implant that surrounds the core part is more rigid than the latter;
- the surrounding shell part is made of one or more materials of which the shear modulus is greater than that of the core part.

The intra capsular lens implant according to the invention aims at physically restoring the flexibility of the crystalline lens and bringing the amplitude of accommodation as close as possible to that of a younger crystalline lens.

The shell part may have a shear modulus less than or equal to the shear modulus of the cortex of the crystalline lens to be physically restored.

To be noted that the core part has a shear modulus less than the shear modulus of the shell part.

Typically, the shell part may have a shear modulus of 5 or 10kPa approximately and the core part may have a shear modulus of 200Pa, 500 Pa, 800Pa, 1000 Pa, or 2kPa approximately. When the shell part is made of one or more of the above materials, the core part may be made of a fluid which therefore acts a softer core part in a more rigid surrounding shell part.

All that has been described above for the intra cortical lens implant of claim 1 also applies to the above intra capsular lens, in particular its core part.

The intra capsular lens implant may also comprise any one of the features defined in dependent claims 2 to 11 and that define possible features of the core part.

Other features and advantages will emerge in the course of the remainder of the description, given by way of non-limiting example only, with reference to the following drawings, in which:
- Figure 1 is a diagrammatic illustration of a human eye in a sagittal cross-section;
- Figure 2 is a diagrammatic illustration of a human eye crystalline lens in a sagittal cross-section;
- Figure 3A is a sagittal view of an intra cortical lens implant according to an embodiment of the invention;
- Figure 3B is a graph representing the variation in the cortical and nucleus shear modulus over time;
- Figures 4A and 4B illustrate different possible intra cortical lens implant shapes in a crystalline lens front view;
- Figure 5 represents a sagittal view of Figure 3A intra cortical lens implant in a crystalline lens;
- Figure 6 illustrates a variant embodiment of an intra cortical lens implant with a non-axisymmetric shape in a sagittal view;
- Figures 7A and 7B schematically illustrate in a crystalline lens CL the extreme volumes that can be occupied by an intra cortical lens implant according to the invention;
- Figures 7C and 7D schematically illustrate in a crystalline lens CL' possible shapes/volumes of an intra cortical lens implant according to the invention;
- Figures 8A-N are views illustrating different steps performed in some of the methods of Figure 9;
- Figure 9 is a flowchart illustrating several possible methods for placing an intra cortical lens implant or an intra capsular lens implant in a patient's eye and for non-invasively treating a patient's crystalline lens;
- Figure 10 is an example embodiment of an apparatus used in different embodiments of the invention;
- Figures 11A-M are views illustrating different steps performed in other methods of Figure 9 for placing an intra capsular lens implant in a patient's eye;
- Figures 12A-F are views illustrating different steps performed in a method for electromagnetically treating a patient's crystalline lens;
- Figure 13 is a schematic view of the main components of a computer system;
- Figure 14 is a schematic view of a window displayed in the graphical user interface of the system of Figure 13;
- Figure 15 is a schematic view of a process implemented by the system of Figure 13;
- Figure 16 is a schematic view of another window displayed in the graphical user interface of the system of Figure 13;
- Figure 17 is a schematic view of another process implemented by the system of Figure 13.

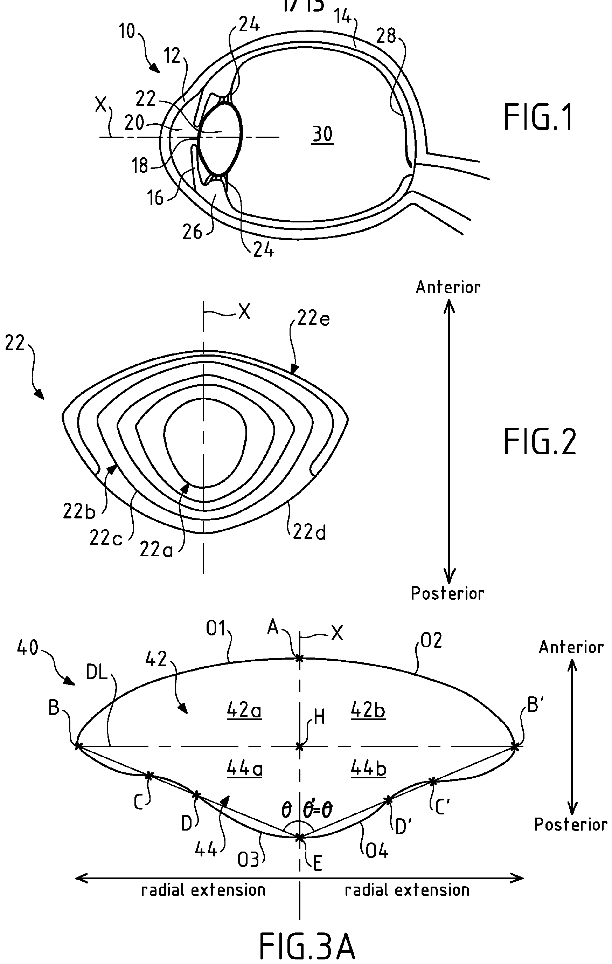

Figure 1 shows a human eye cross-section (viewed in a sagittal plane) or side view.

The eye 10 includes the cornea 12 and the sclera 14. The iris 16 and pupil 18 are located behind the cornea 12. The iris includes muscles called sphincter muscles that are able to contract and expand in order to control the diameter of the pupil 18.

The eye includes an anterior chamber 20 that is located between the cornea and the iris and pupil.

A crystalline lens 22 is located posterior to the cornea 12 and the iris 16. Crystalline lens 22 is sustained by zonules 24 which form a complex web connecting the latter to the ciliary muscle 26. Thanks to this sustaining configuration the lens is able to change shape for focusing.

The eye also includes the retina 28 located at the back of the eye, the purpose of which is to form an image of an object looked at by the eye.

A posterior chamber 30 located between crystalline lens 22 and retina 28 forms the major part of the eye and is filled in by the vitreous body not represented here for the sake of clarity.

The optical axis of the eye is commonly known as being an average between the optical axis of the cornea and the optical axis of the crystalline lens. In Figure 1 an optical axis X is illustrated and will be used in the remainder of the description as being the optical axis of the crystalline lens.

Figure 2 illustrates a diagrammatic overall enlarged view of crystalline lens 22 in a sagittal plane. Such a plane includes the optical axis X of the lens.

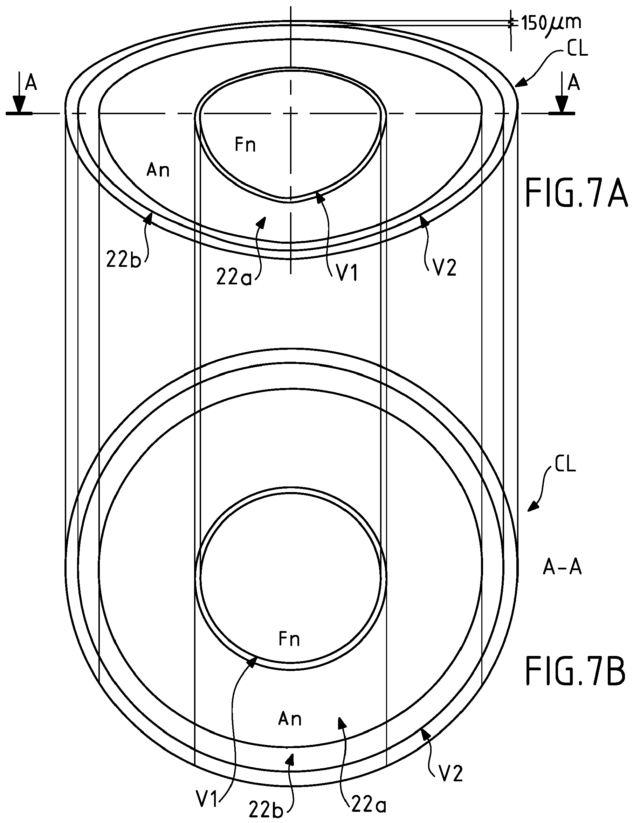

Crystalline lens 22 is a multilayered structure of collagen and crystalline that can change its optical power depending on its state of deformation. The lens has an onion-like structure and comprises four sub-parts or components:
- the nucleus 22a is a central part made of multiple layers, also known as age related nuclei; in particular, nucleus 22a successively includes for an adult, from the center towards the periphery, the embryonic nucleus (En), the fetal nucleus (Fn), the juvenile or infantile nucleus (Jn) and the adult nucleus (An) which are not identified on Figure 2 for the sake of clarity (some of these different layers are illustrated in Figures 7A-D);
- the cortex 22b surrounds nucleus 22a and the frontier between both sub-parts is denoted 22c;
- the capsule 22d is a thin, relatively stiff bag that contains both nucleus 22a and cortex 22b;
- the epithelium 22e is an anterior layer located in between the anterior portion of cortex 22b and capsule 22d (also called capsular bag) and made of active cells.

The external shape of the lens looks like an ellipsoid in a sagittal plane, with a greater curvature on the posterior pole than on the anterior one.

The inventors have discovered that appropriate flexibility can be restored to a crystalline lens which hardens with age (presbyopia is the first optical disorder that appears in connection with the aging of a lens). Accommodative amplitude of an aged natural lens can therefore be modified, in particular increased, so as to provide the patient with comfort visual accommodation. The patient's eye is therefore restored with visual accommodation of a younger eye. This has been made possible thanks to new lens accommodative devices, in particular specific implants, and methods/systems for implementing/achieving these lens accommodative devices in patient's eyes, in particular the specific implants. The lens accommodative devices, in particular specific implants, in accordance with the present embodiments have not anatomical outlines or inner structures. However, in some other embodiments they may have anatomical outlines or inner structures. The inventors have also discovered that new lens physical restoration methods which do not involve any implant make it possible, when applied to a patient's crystalline lens, to restore flexibility and, therefore, increase accommodative amplitude. These methods may restore optical properties to the crystalline lens and possibly, when needed, bring optical correction(s), e. g. correct astigmatism and increase the depth of field.

Figure 3A illustrates an intra cortical lens implant according to a first embodiment of the invention.

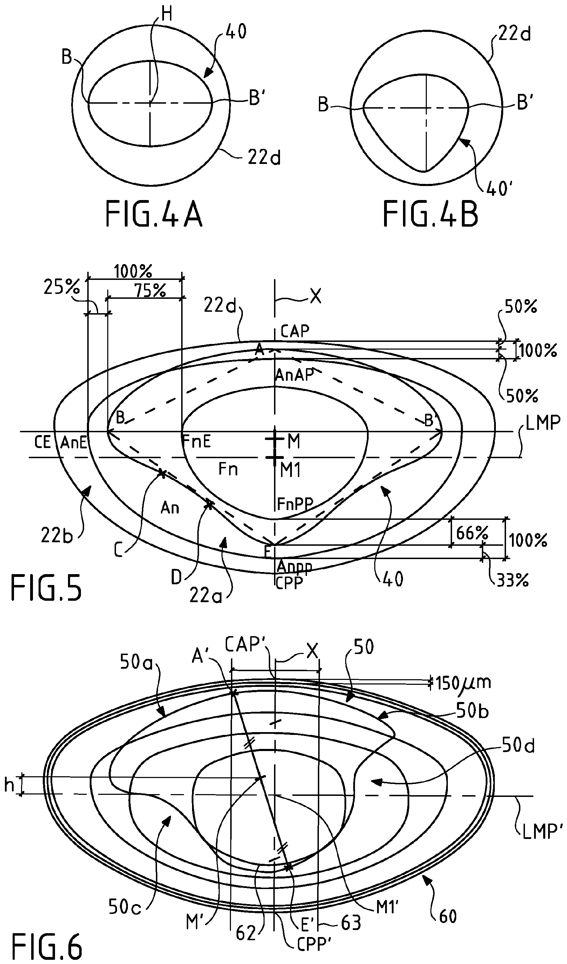

This view illustrates an intra cortical lens implant 40 in a sagittal plane which has an axi-symmetrical shape relative to its longitudinal axis, also called polar axis as it passes by the poles of the implant as will be seen subsequently. This means that each portion located on either side of the axis has the same shape as in a mirror whatever the cross section plane including this axis. When such an implant 40 is placed within the Figure 2 lens it is centered about the optical axis X of the lens and the polar axis of the implant 40 therefore coincides with the optical axis X of the Figure 2 lens. For this reason the polar axis of the implant 40 is considered as identical to the optical axis X of the lens. Thus, the axis X is considered as both the optical axis of the lens and the polar axis of the implant in the remainder of the description unless otherwise specified such as in the description of Figure 6 where the polar axis of the implant does not coincide with axis X.

The intra cortical lens implant 40 has for instance an overall shape of a mushroom or butterfly wings.

This implant is intended to be located within crystalline lens 22 (Figure 2) at a location which will be described subsequently. When in place the implant 40 will occupy a portion of the inner volume (this volume comprises nucleus 22a and cortex 22b) of the crystalline lens 22 that is bounded by the capsular bag 22d. This holds true for any other shape of intra cortical lens implant envisaged in accordance with the present invention.

As illustrated in Figure 3A, the implant 40 extends axially along its polar axis from an anterior location represented by a point called anterior pole A to a posterior location represented by a point called posterior pole E. Poles A and E are fixed whatever the sagittal cross section of the implant.

Implant 40 also extends radially relative to its polar axis, i.e. in a direction that is perpendicular to this axis.

Although the intra cortical lens implant is represented in a sagittal plane it is a 3D volume and its radial extension is to be considered in every radial direction around its polar axis X perpendicularly thereto, e.g. as illustrated in the front view of Figure 4A.

As illustrated in Figure 3A, the radial extension of implant 40 varies along the polar axis when following the outer outline of the implant from anterior pole A to posterior pole E: it first increases when following the outer outline from anterior pole A to both points B and B' simultaneously (maximum radial extension) and then decreases when following the outer outline from points B and B' to posterior pole E simultaneously. The points B and B' in a sagittal section are located on the equatorial plane of the implant which is here represented by the dashed line DL (this line is the intersection between the equatorial plane and the sagittal plane). This definition of the equator also applies to other inner structures/layers of the lens.

The implant 40 comprises a body in a solid form or state, i.e. having a given shape that can be maintained as such in the absence of any external constraints (such as constraints exerted by an outer device). It therefore has cohesive properties.

As will be seen subsequently, the intra cortical lens implant may alternatively take other embodiments forms.

The body is made of one or more materials with elastic or visco-elastic properties which have a shear modulus that is greater than 10 Pa and less than 10 kPa. This guarantees that the intra cortical lens implant will be flexible enough relative to the remainder of the natural crystalline lens that surrounds the implant and that is more rigid than the latter. As already mentioned above the one or more materials of the implant are such that it can be elastically deformed. The one or more materials may be a gel. The value of the shear modulus is adapted to the patient's eye and particularly the crystalline lens, i.e. its mechanical properties (according to clinical results or literature) and geometrical features (obtained through imaging such as the residual amplitude, i.e. the gap between the most and the least accommodated position of the lens, the geometry specific to each position, i.e. for each position the anterior and posterior curvature radii, the equatorial diameter and the thickness or axial extension of the lens along the optical axis X, and the possible axial shift of the lens between the two extreme positions).

Typically, the natural peripheral part of the crystalline lens that surrounds the intra cortical lens implant has a shear modulus greater than 1 or 2 kPa (which corresponds to a 40 years eye on average), which corresponds to a more rigid or stiff part than the implant.

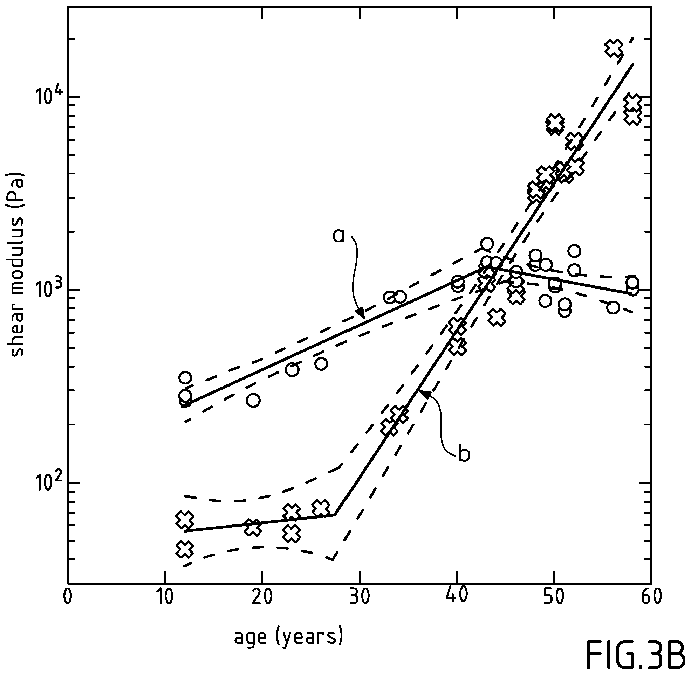

Figure 3B represents the evolution of the shear modulus (it is a logarithmic scale of the shear modulus) of both the cortex (curve a) and the nucleus (curve b) of an eye over time between ages of 10 and 60. These curves are illustrated and described in an article entitled "Shear modulus data for the human lens determined from a spinning lens test" from Wilde GS, Burd HJ, Judge SJ, Experimental Eye Research, vol. 97 Issue 1, p 36-48, April 2012. These curves show an inversion area between 40 and 50 years around a shear modulus value of 1kPa: the shear modulus of the nucleus (curve b) keeps on increasing with age with a greater rate than the shear modulus of the cortex (curve a) and with greater values. Past this inversion area the relative properties between the nucleus and the cortex are inverted. The curves illustrate the trend that has been observed on a plurality of patients' eyes over years (this is an example of statistical clinical data). Thus, for a given patient affected with presbyopia the shear modulus of the intra cortical lens implant will be selected at a value much less than that of the natural cortex. This will make it possible to get back to a more favorable zone of the graph, prior to the inversion area, where the nucleus is younger and softer. For example, for a cortex with a shear modulus value of 1 kPa the nucleus will be allocated with a value of 0.5 kPa.

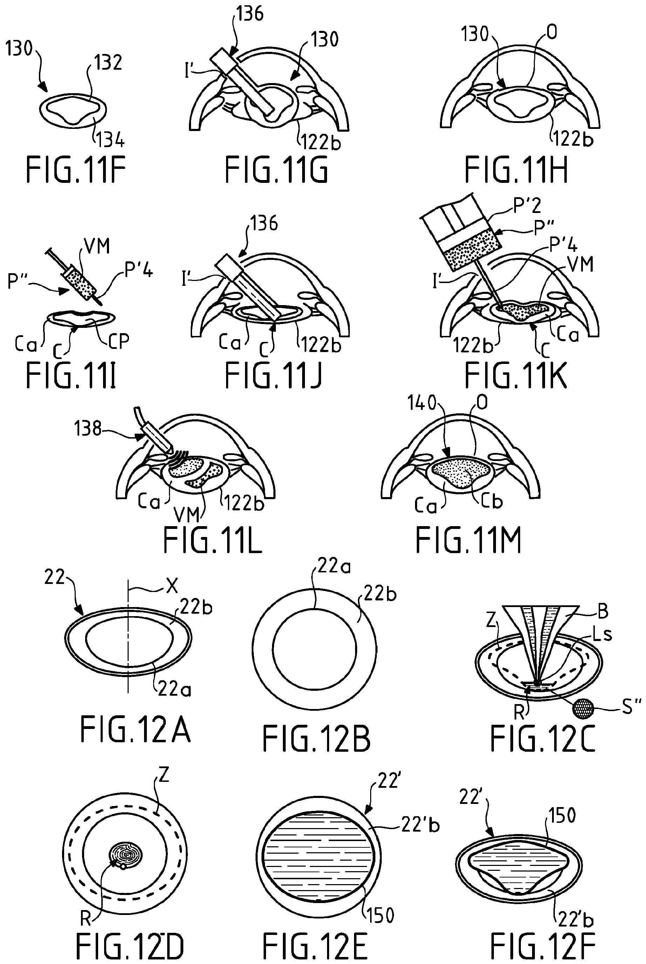

In another embodiment such as that illustrated in Figures 11F-H the peripheral or surrounding implant part 134 (shell part 134) may be slightly softer than the initial (natural) cortex and may take a shear modulus value of 0.7 kPa for example, or may have a viscosity in register with this shear modulus. The nucleus may take a shear modulus value of 0.5 kPa for example, or may have a viscosity in register with this shear modulus.

By way of example, the material body is made of silicon or hydrophilic acrylics or hydrophobic acrylic or elastomer and, for example, may be cross-linked polydimethylsiloxanes possibly reinforced with silica or EMA (ethyl methacrylate), or HEMA (Hydroxyethyl methacrylate), or a combination of both previous examples (co-polymers) (material adapted for a medical use). Other materials may alternatively be used for manufacturing the intra cortical lens implant.

The implant 40 comprises an anterior part 42 and a posterior part 44 that are each defined along the polar axis X by extending there along.

The anterior part 42 begins at anterior pole A and extends rearwardly towards the posterior part. The anterior part 42 is defined by the upper part of the body (Figure 3A) that is delineated by the outer convex outline that connects point B to point B' through anterior pole A and by the equatorial plane (line DL) that connects directly point B to point B'. Put it another way, the anterior part 42 is located on one side of the equatorial plane (upper side in Figure 3A) and the posterior part 44 is located on the opposite side (lower side in Figure 3A).The point denoted H that is situated both on the equatorial plane and axis X corresponds to the orthogonal projection of each of points B and B' on axis X.

As already mentioned above, the anterior part 42 extends radially relative to the polar axis X on either side thereof in the sagittal plane of Figure 3A.

On this sagittal plane of Figure 3A, the anterior part 42 has two portions located on both sides of the longitudinal axis respectively: a left side portion 42a and a right side portion 42b that are symmetrical to each other with respect to axis X in this embodiment.

To be noted that in other embodiments the left and right side portions of the anterior part may not be symmetrical.

Each portion 42a, 42b of the anterior part has a radial extension or dimension that increases from the anterior pole A to point B for portion 42a and to point B' for portion 42b, where the anterior part 42 ends and the posterior part 44 begins.

The outer convex outline O1, 02 of each portion 42a, 42b forms a continuous curve in the plane of Figure 3A with a radius of curvature that is greater at the anterior pole A than at point B and B' depending on the portion.

In the present embodiment the radius of curvature continuously decreases from A to B for left side portion 42a and from A to B' for right side portion 42b. This configuration of the anterior part of the intra cortical lens implant makes it possible to continuously increase the thickness of the anterior part of the crystalline lens that is located between the capsular bag and implant 40 (see for example Figure 5). This may therefore cause a continuous and controlled increase in the rigidity of this anterior part of the crystalline lens. This configuration makes it possible to precisely control the distribution of the stresses between the ciliary muscle and the zonules and the softer/more flexible intra cortical lens implant through the peripheral more rigid part of the crystalline lens (cortex).

However, in other embodiments, the radius of curvature may locally vary differently between A and B (same between A and B'), e.g. by successively alternating portions of curve with decreasing and increasing radii of curvature such as waves while overall decreasing from A to B (same from A to B'). This alternate configuration provides an overall control increase in the rigidity of the lens anterior part. Such an alternate configuration with local curvatures induces controlled astigmatism and increase in the depth of field. Also, such a shape makes it possible to improve spherical aberrations that disturb visual acuity by creating a gradient of refractive index depending on the refractive index of the material.

As represented in Figure 3A, the value of the angle θ defined by the two segments [A, E] and [B, E] in the sagittal plane may be comprised between 1° and 90°. This is the same for the angle θ' (here the two angles θ and θ' are the same) between the segments [A, E] and [B', E]. Such an angle makes it possible to have an intra cortical lens implant with sufficient flexibility or softness relative to the more rigid part of the crystalline lens located around the intra cortical lens implant within the capsular bag (ex: cortex). More particularly, such an angle θ makes it possible to control the curvature change of the anterior face of the crystalline lens in accordance with the direction and the distribution of the stresses that are available in a conventional zonular system.

However, in other configurations, the angle may differ from one side to the other (θ ≠ θ') when the intra cortical lens implant has not an axi-symmetrical configuration (ex: Fig 6).

As already mentioned above, the posterior part 44 extends radially relative to the polar axis X on either side thereof in the sagittal plane of Figure 3A.

On this sagittal plane of Figure 3A, the posterior part 44 has two portions located on both sides of the longitudinal axis respectively: a left side portion 44a and a right side portion 44b that are here symmetrical to each other with respect to axis X in this embodiment.

Each portion 44a, 44b of the posterior part has a radial extension that decreases from the point B for portion 44a and from the point B' for portion 44b respectively, where the posterior part begins, towards the posterior pole E.

As represented in Figure 3A, the outer outline 03, 04 of each portion 44a, 44b respectively is not a straight line between B and E (resp. B' and E).

More particularly, each outer outline 03, 04 forms a continuous curve between the point B, B' respectively and the posterior pole E in Figure 3A. Each curve is wavy in this embodiment.

Each outer outline 03, 04 includes two points C, D, respectively C', D', located on the curve between the point B, respectively B', and the posterior pole E and that form two inflexion points for the curve.

As represented in Figure 3A, the two inflexion points C, D, respectively C', D', are located on a straight line between the point B, respectively B', and the posterior pole E.

Each curve between the point B, respectively B', and the posterior pole E includes two side portions (B, C), respectively (B', C'), and (D, E), respectively (D', E), flanking a central portion (C, D), respectively (C', D'). The two side portions for each curve have a curvature of the same type and the central portion has a curvature of the opposite type.

Each curve might therefore be seen here as including a double hump.

Thus, in the present embodiment, the two side portions (B, C) and (D, E) for curve 03 (respectively (B', C') and (D', E) for curve 04) have an outwardly convex shape while the central portion (C, D) (respectively (C', D')) has an inwardly concave shape.

To be noted that the overall shape of each curve 03 and 04 can be inverted in a variant embodiment: the two side portions are concave and flank a central convex portion. Alternatively, only one shape of a curve is inverted relative to the other curve.

This overall shape is adapted to a patient and here makes it possible to create an aspherical intra cortical lens implant:
- the region corresponding to DED' has a marked curvature allowing close objects to be seen;
- the region corresponding to BC and B'C' has a less marked curvature which allows far objects to be seen.

Overall the shape of the curve connecting B and E as well as B' and E is a polynomial of a degree that is adapted to the patient's eye. This polynomial may be designed so as to favor the volume of nucleus to be treated relative to the untreated volume of cortex in the natural crystalline lens with a view to obtaining a spring effect. This spring effect relies on the more rigid cortex on which the intra cortical lens implant may rest and compress before returning to its initial shape.

To be noted that in any sagittal cross-section the four points A, B, E and B' form a quadrilateral and the outer outline of the posterior part has to be convex overall even though some local concavities may be present. This overall biconvex shape of the anterior part and posterior part outer outlines makes it possible to have a biconvex intra cortical lens implant. When the quadrilateral ABEB' is defined the four points are properly defined relative to each other. The two segments AE and BB' intersect and are perpendicular to each other in the implant 40. However, in a non axi-symmetrical shape the segments AE and BB' are not perpendicular to each other. In such a configuration the two poles A and E are still the same whatever the sagittal plane (passing by the polar axis AE) whereas the segment BB' may vary depending on the sagittal plane.

The above holds true for any other shapes of intra cortical lens implant according to the invention than that of Figure 3A and for instance for non-axi symmetrical implant shapes.

If the posterior part were concave overall then optical artefacts, diffraction might occur and the gradient of refractive index (GRIN) would vary in an undesired way. In addition, the mechanical performance of such a soft intra cortical lens implant on the crystalline lens could be adversely affected due to a less efficient mechanical cooperation between the concave posterior part of the intra cortical lens implant and the more rigid posterior cortex against which it rests.

It is to be noted that the two side portions of each curve here are symmetrical to each other relative to the polar axis of the implant.
In another embodiment the two side portions may not be symmetrical to each other relative to the polar axis of the implant, and the angles θ and θ' may be different between the two side portions. Such an asymmetrical configuration may be useful, e.g. when one of the patient's eyes is affected by a strong astigmatism. In such a case, an optical correction is applied through an asymmetrical configuration of the intra cortical lens implant in order to create a deformation of the intra cortical lens implant that is different from one side to the other when installed in the natural lens.
The intra cortical lens implant adapts to the patient's eye morphology and may therefore be symmetrical or asymmetrical depending on the morphology, with different anterior and posterior portions than those of Figure 3A (however, these portions keep the same main characteristics in terms of convexity of the anterior and posterior parts and opening angles θ and θ').
In other embodiments the curvature radius of one or several of the side and central portions of one or both curves 03 and 04 may vary (with respect to the curvatures of Figure 3A) so that the convexity/concavity is more or less pronounced. In other alternative embodiments the number of convex and/or concave portions may differ between left and right curves relative to the polar axis of the implant.

Whatever the embodiment, the posterior part outer outline may be continuous and convex overall so as to form an optical interface adapted to the patient's eye morphology.

To be noted that the shape of the posterior part outer outline may vary in other embodiments that are not depicted here.

By way of example, the outer outline between B and E (same for B' and E) may be strictly convex or rectilinear and the connection or edge between the outer outline and the anterior part at point B (same for B') is rounded. The shape of the outer outline at point E is also rounded. If any sharp edge were present on the optical path or at any point on the outer outline of the intra cortical lens implant, then unwanted mechanical stresses and optical artefacts and diffraction might be generated during the implant deformation in situ.

Whatever the accurate shape of the intra cortical lens implant, its overall aim is to restore mechanical properties, in particular flexibility, to the natural crystalline lens when implanted so as to increase accommodative amplitude and, therefore, at least reduce presbyopia disorders. Such an intra cortical lens implant may also provide optical correction(s) for correcting astigmatism and/or increasing the depth of field in the patient's eye.

Figure 4A illustrates a front view, i.e. a view taken in a plane that is perpendicular to the polar axis of the implant (here axis X), of the implant 40 located inside capsular bag 22d. This view is taken in the equatorial cross-section plane perpendicular to a sagittal plane and that includes points B and B' of Figure 3A.

As represented in Figure 4A, the cross section shape of the intra cortical lens implant is that of an ellipse.

However, in other embodiments not represented here, the outer outline of the intra cortical lens implant in a front view plane as that of Figure 4A may assume other shapes, e.g. with recesses and bumps, or a perfect circle.

Figure 4B illustrates a front view of an intra cortical lens implant 40' according to a variant embodiment located inside capsular bag 22d. The implant 40' has the same overall features as implant 40 of Figure 3A except that the left and right side portions of the intra cortical lens implant relative to the polar axis of the implant, and thus, points B and B', are not symmetrical to each other. The intra cortical lens implant has therefore not an axi-symmetrical shape relative to its longitudinal axis when viewed in a front plane. Here the polar axis of the implant does not match the optical axis X of the lens.

As shown in Figures 4A and 4B embodiments, the intra cortical lens implant can take various forms in cross sectional planes that are perpendicular to the polar axis of the implant and to any sagittal plane thereof. As represented in Figure 4B, the radial extension of the implant 40' is not the same in every radial direction.

Figure 5 is a sagittal cross section of the intra cortical lens implant 40 inside the capsular bag 22d of the crystalline lens and surrounded by a remaining natural part of the lens. The intra cortical lens implant occupies a part of the lens that has been previously removed as will be seen subsequently

Several salient geometrical and physiological points/zones have been identified in the sagittal cross section of the lens as follows:
- CAP designates the anterior pole of the crystalline lens that is located on the optical axis X (as already mentioned above, here the polar axis of the implant coincides with the optical axis of the lens X);
- CE designates the lens equator (this is a physiological zone surrounding the lens);
- AnE designates the adult nucleus equator;
- FnE designates the fetal nucleus equator (this is a physiological zone surrounding the embryonic nucleus);
- AnPP designates the adult nucleus posterior pole;
- CPP designates the cortex posterior pole;
- FnPP designates the fetal nucleus posterior pole; and
- AnAP designates the adult nucleus anterior pole.

FnE is represented on the contour of the fetal nucleus (at the location it would occupy if the nucleus were present in the lens) and the same holds true for FnPP.

In an embodiment, these above geometrical and physiological points/zones may satisfy the following simple mathematical relations that are also illustrated in Figure 5 for intra cortical lens implant 40:
- [A, AnAP] = ½ [CAP, AnAP] (1)
- [B, FnE] = ¾ [AnE, FnE] (2)
- [E, FnPP] = 2/3 [AnPP, FnPP] (3).

When relation (1) is satisfied this means that there is a sufficient anterior distance and therefore a sufficient thickness of a more rigid part between the soft implant 40 and the capsular bag 22d so that sufficient relative rigidity is achieved between the non treated lens (mainly the cortex) and the intra cortical lens implant in the anterior part of the lens.

This sufficient thickness of the anterior rigid layer or shell surrounding the intra cortical lens implant makes it possible to redirect the stresses from the zonules towards the central zone of the lens, i.e. the implant. This relation may also hold true for other intra cortical lens implant shapes.

A minimal distance above 150 µm between any point of the outer shape of the implant 40 (or any other implant according to the invention) and the lens external capsule profile ensures that the active cells of the epithelium will not be damaged by the presence of the implant.

When relation (2) is satisfied this means that there is a sufficient equatorial rigid transmission zone around the intra cortical lens implant since the zonules are connected to this zone (fig. 5) and therefore exert a mechanical tension or stress thereon during the accommodative process.

As represented in Figure 5, a soft intra cortical lens implant is surrounded by a more rigid part of the natural crystalline lens. The softness/flexibility of the implant enables its compression when the zonules exert a tautness on the lens, in particular in the central zone of the implant that would correspond to the nucleus. This is because the rigid/stiff posterior part of the natural (genuine) crystalline lens that is located between the posterior part of the intra cortical lens implant and the capsular bag serves as a rigid basis for the implant in order to give rise to a spring effect.

Depending on the shape of the intra cortical lens implant the axial extension of the rigid part located behind the posterior part of the implant may be more or less great provided that it is sufficient for producing the above effect. In other words the posterior part of the natural crystalline lens has to keep its structural stiffness and appropriate mechanical reaction as much as possible in order to round the lens when releasing zonules 24 for near vision. According to above relation (3), a sufficient volume of posterior natural tissues remains in the lens to make it possible for the intra cortical lens implant to produce such a spring effect.

The shape of the soft/flexible anterior part of the intra cortical lens implant enables a change in the outer curvature of this anterior part when the zonules exert a tautness on the lens.

More particularly, as explained in relation with Figure 3A, the shape of the outer outline O1 and O2 in the anterior part of the intra cortical lens implant causes an increase in the thickness of the rigid/stiff posterior part of the natural crystalline lens when moving from anterior pole AP towards the periphery of the lens. This makes it possible to control the quadratic moment of the shape and, therefore, maximize the transmission of the zonular effort and appropriate consecutive central deformation of the lens (flattening).

All that has just been mentioned above in relation with Figure 5 (in particular as regards the technical effects and functions) equally applies to any other shape of implant according to the invention.

Generally speaking the intra cortical lens implant 40, as well as any other implant according to the invention, is mainly located in the anterior part of the lens so as to give rise to the above-described technical effects. This is illustrated, by example, in Figure 5 by the relative position between the intra cortical lens implant 40 and the lens. M is the mid-point located on the segment that connects the anterior pole A and the posterior pole E of the implant and LMP represents the lens plane that is perpendicular to the polar axis (optical axis X) and crosses the latter at point M1 that is midway the lens poles CAP and CPP . Thus, mid-point M is located above plane LMP in Figure 5, i.e. it is located in the anterior part of the lens. This explanation holds true for any other implant according to the invention.

To be noted that the outer outline of any implant according to the invention must not have sharp angles but a smooth shape so as to best mechanically cooperate with the surrounding crystalline lens and avoid to cause any injury thereto. Local stresses and optical artefacts can thus be avoided.

Non-axisymmetry of the intra cortical lens implant in some embodiments may induce desired optical features (ex: desired astigmatism) such as a controlled increase in the depth of field.

Overall the implant according to the invention (whatever its embodiments/shapes) gives rise to a new accommodative amplitude that makes the lens flatter than it is for far vision when submitted to zonules constraints (decrease in curvature) and elastic enough to come back to a rounder shape when ciliary muscle contracts. The thus obtained rounder shape is equivalent to the previous possible roundest shape (obtained for the lens without the implant) but might go beyond the latter in some instances.

Thus, a new offset is needed for this new optical system. This offset could be reached through any kind of stromal refractive surgery e.g. by changing the curvature of the cornea or by creating internal optical diopters in the same modified lens material/zone.

Figure 5 illustrates the four points A, B, E and B' of the intra cortical lens implant forming a quadrilateral in dashed lines that is here inscribed within the outer shape of the intra cortical lens implant 40. This quadrilateral may also be present in any other intra cortical lens implant according to the invention and the shape of the implant develops around or is based on this quadrilateral.

Figure 6 illustrates a variant embodiment of a non axi-symmetric intra cortical lens implant 50 inside a crystalline lens 60 in a sagittal plane including optical axis X of the lens. Here the polar axis A'E' of the implant 50 does not coincide with optical axis X of the lens. The intra cortical lens implant 50 has two left and side portions 50a and 50b that are not symmetrical to each other relative to the polar axis A'E' for the anterior part of the intra cortical lens implant as well as for the posterior part thereof. The right side portion is smaller than the left side portion and, more particularly, the right side portion has an anterior part that has a smaller radial extension than the left side.

Further, the posterior part 50c, 50d of each of left and side portions 50a and 50b is dramatically asymmetrical since:
- the outer outline of the right side portion 50b makes a smaller angle to axis X than that of left side portion 50a;
- the curvatures of the different portions of each of the left and right side portions posterior part are inverted and there are not as many portions in both left and right side portions: for left side portion 50a, two outwardly convex shapes flank a central concave portion whereas, for right side portion 50b, there are only a convex portion and a concave adjacent to each other.

Such an asymmetry causes astigmatism and therefore an increase in the depth of field.

The intra cortical lens implant covers here completely the fetal nucleus 62 of the lens.

Such an asymmetrical shape enables the suppression of mechanical blocking of the lens accommodative mechanism which is due to the natural increase in the relative rigidity of the nucleus with age.

Overall the intra cortical lens implant according to the invention is tailored to the patient's eye and, e.g. may be adjusted either for correcting astigmatism and/or for compensating a non desired deformation in the lens.

To be noted that the main features of this implant 50 correspond overall to that of implant 40, in particular, as regards the anterior part of each left and side portion and their posterior part and the relative position of the intra cortical lens implant in the lens, the quadrilateral structure ABEB' etc.. The shapes are however somewhat different as well as the values for the angles, curvature radii, radial extension etc.

In addition, Figure 6 also illustrates another possible configuration that better defines the location of an intra cortical lens implant inside a lens.

In the present embodiment the anterior pole A' and the posterior pole E' of the implant are located inside an infinite cylinder (or at least a cylinder that is as long as crystalline lens thickness or height on Figure 6) 63 centered about axis X and having a diameter of approximately 2 mm. Here the polar axis A'E' of the implant intersects optical axis X. However, this may not be the case in other configurations where both axis may be parallel to each other.

The above may apply to any other implant according to the invention and helps to better define the location of an intra cortical lens implant inside a lens whatever the shape of the latter and its axi- or non-axi- symmetrical shape. Such a definition also contributes to the overall technical effects of the intra cortical lens implant, e.g. as those already described above in relation with Figures 3A and 5.

Further, here M' is the mid-point located on the polar axis and LMP' represents the lens plane that is perpendicular to the optical axis X and crosses the latter at point M'1 that is midway the lens poles CAP' and CPP'. Thus, mid-point M' is located above plane LMP' in Figure 6 (at a distance h therefrom), i.e. it is located in the anterior part of the lens, for the same reasons as in Figure 5.

Figures 7A and 7B schematically illustrate in a crystalline lens CL (in a sagittal plane and in a front plane viewed in a cross section according to AA) the extreme volumes V1 and V2 that can be occupied by an implant according to the invention.

Overall the volume of the intra cortical lens implant according to the invention (whatever its shape and location) is between 10% and 90% of the crystalline lens inner volume (these values may be taken) so as to provide the lens with appropriate elasticity or flexibility.

A minimal volume V1 of 10% approximately corresponds to the volume of the fetal nucleus (Fn).

A maximal volume V2 of 90% approximately encompasses the whole nucleus 22a and may even encroach locally upon the cortex 22b.

These extreme volumes/sizes represent respectively the minimal and maximal overall functional zones to be treated but do not identify the accurate geometries and relative positions of the intra cortical lens implants that will occupy each of these zones or zones in-between. The intra cortical lens implant geometries may somewhat differ from the overall geometry of the functional zones.

Overall, the intra cortical lens implant may take various forms the volume of which lies between 10% and 90% of the crystalline lens inner volume. However, the outer shape of the intra cortical lens implant must not locally be too close to the epithelium as already described with reference to Figure 5 (not at less than 150 µm from the external capsule profile).

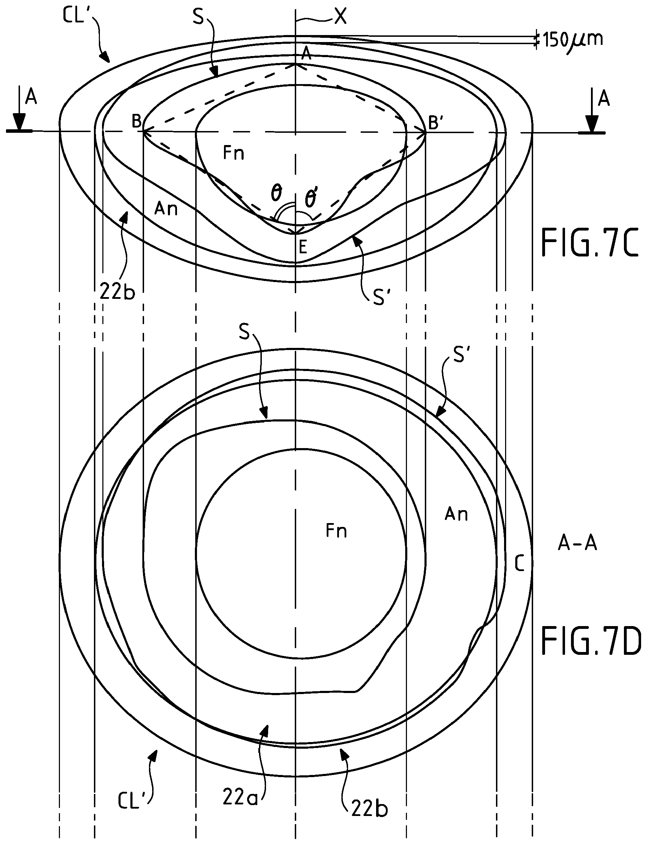

Figures 7C and 7D schematically illustrate in a crystalline lens CL' (in a sagittal plane and in a front plane viewed in a cross section according to AA) possible intra cortical lens implant shapes and volumes according to the invention. Again the quadrilateral formed by the four points A, B, E and B' has been represented.

For example, the intra cortical lens implant S in solid lines represents a possible implant shape corresponding to a functional zone located in between the two extreme volumes. The implant S has a volume of approximately 20% of the crystalline lens inner volume. Implant S does not wholly cover fetal nucleus (Fn) but a major portion thereof and here the whole anterior part of fetal nucleus is covered. Only small posterior portions of the fetal nucleus are not covered. This shape evolves around the fetal nucleus shape. Implant S is not axi-symmetrical relative to axis X (and to its polar axis) and angles θ and θ' for the two side portions of the intra cortical lens implant in Figure 7C are different from each other.

The outer outline of intra cortical lens implant shape S includes curvatures and rounded edges that more precisely define an appropriate shape tailored to a patient's eye. This shape may be the result of the use of a simulation model together with clinical measurements performed on the patient's eye as will be further described.

Whatever the accurate shapes the intra cortical lens implant may assume it has to occupy a part of the natural crystalline lens that includes at least a major portion of the fetal nucleus so as to restore flexibility in this core zone relative to a more rigid/stiffer surrounding zone of the untreated natural lens. The flexibility restoration in this zone of the lens is necessary to allow the lens shape to be deformed as desired during the accommodative process.

In some instances, the juvenile and adult (An) layers of the nucleus may also be at least partly impacted/covered by the intra cortical lens implant as represented by the intra cortical lens implant S.

In Figures 7C and 7D another shape S' represents a possible intra cortical lens implant shape with a volume of approximately 60% of the crystalline lens inner volume that locally covers an anterior portion of the cortex. Implant S' also covers the major portion of adult nucleus (An). The 60% volume value of the total crystalline lens inner volume represents a preferred value in terms of technical effects according to the invention.

When the intra cortical lens implant encroaches upon the cortex there should not be excessive overlap with the anterior and posterior cortex so as to keep the functionalities described above (sufficient thickness of rigid anterior and posterior cortex for the distribution of stress and spring effect etc.).

For instance, the intra cortical lens implant of Figure 5 occupies 50% of the lens inner volume and 35% for the Figure 6 intra cortical lens implant.

All that has been described above in relation with Figures 3A, 5 and 6 may also apply here to intra cortical lens implants that occupy the extreme functional volumes/zones as well as intermediate volumes/zones, in particular, regarding the anterior part of the crystalline lens and the posterior rigid part of the natural crystalline lens, and more generally, regarding the relative position of the intra cortical lens implant to the lens (main location in the anterior part of the lens, polar axis within a given cylinder etc.).

Given the implant volumes requirements explained above, the angles θ and θ' illustrated in Figure 3A may each lie between 1° and 90° as explained above for an axi-symmetrical intra cortical lens implant shape (all the sagittal cross sections are the same and θ= θ').

If the angle θ is less than 1°, the intra cortical lens implant will be too small to produce a mechanical effect on the crystalline lens and the flexibility brought by the intra cortical lens implant will therefore not provide the lens with enough accommodative amplitude.

Conversely, if the angle θ is greater than 90°, the intra cortical lens implant will be less efficient since either a too large portion of cortex will be occupied by the implant or a too small portion of nucleus will be affected by the implant. The spring effect will therefore disappear.

More particularly, the above angle may be comprised in a narrower range lying between 15° and 75° to provide better results (the intra cortical lens implant thus covers a greater zone of the lens including the equatorial diameter and better manages the distribution of stresses in the implanted lens).

Still more particularly, the above angle may be comprised in a still narrower range lying between 30° and 60° to provide even better results and, more preferably, between 45° and 60°. An angle of 60° is an example in the present embodiment.

The above discussion regarding the values of the angles θ and θ' applies equally if the intra cortical lens implant has not an axi-symmetrical configuration.

However, for a non axi-symmetrical configuration other aspects may be envisaged for the angles θ and θ' since it has to be kept in mind that the implant volume has to be overall between V1 and V2 (these values may be included) as explained above.

In particular, in one or more given sagittal sections the angles θ and θ' may be both (or only one of them) locally either small, e.g. less than 1° or great, e.g. greater than 90° provided that the overall implant volume meets the above requirements. In other words, the angles have to be properly balanced all around the polar axis so as to ensure a minimal zone for the implant.

According to another possible configuration, if, in one or more sagittal sections, the angle θ has a value of 1° or of a few degrees, then the other angle θ' may have a greater value provided that the overall implant volume meets the above requirements.

According to still another possible configuration, the value of 90° for the angles θ and θ' is preferably not reached at the same time in one and the same sagittal section (for the two side portions of the implant in this section). In this respect, if one angle takes the value of 90°, then the other angle is preferably less than 90° so that the posterior part still takes a convex shape overall and a quadrilateral ABEB' be still present in the implant shape.

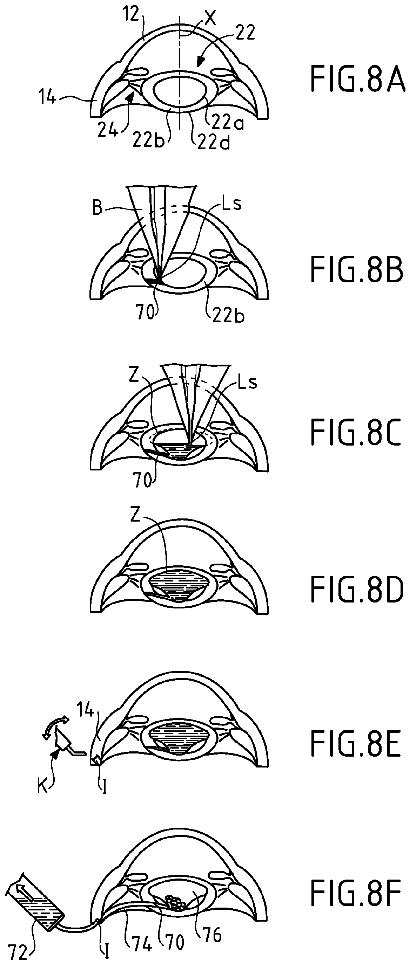

Figures 8A-N schematically represent different views of methods steps for putting in place intra cortical lens implants according to embodiments of the invention in a crystalline lens such as any one of the implants described above. By way of example, the intra cortical lens implant 40 will be considered for one embodiment (Figs 8A-J).

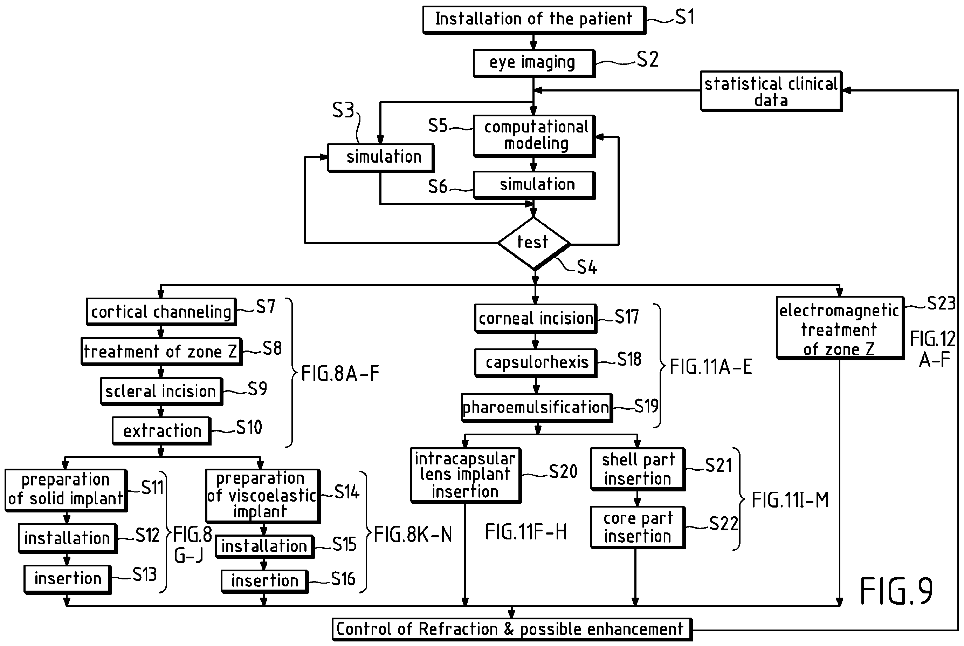

An example embodiment of possible methods is illustrated in Figure 9.

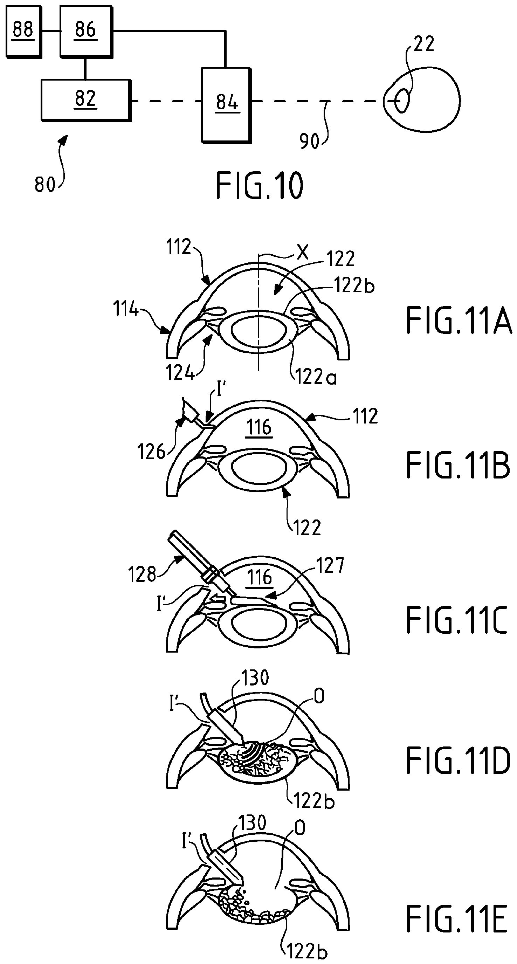

An example of an appropriate apparatus and associated equipment for implementing at least several steps of these methods will be described further with reference to Figure 10.

Figure 8A illustrates in a sagittal plane the eye 10 of a patient (as in Figure 1) with a natural crystalline lens affected with presbyopia disorders such as the one described with reference to Figure 2. For the sake of simplicity, only the nucleus 22a surrounded by the cortex 22b have been represented inside the capsular bag 22d in Figure 8A.

The methods illustrated in Figure 9 includes several steps that spread over time. The steps may be grouped in three phases:
- a first conventional imaging phase during which measurements and observations relating to a patient are performed and a diagnosis may also be made based on these previous results and data (steps S1 and S2);
- a second phase during which mechanical and visual simulation is performed based on the previous phase results (steps S3 to S6);
- and a third phase in the course of which a patient's crystalline lens is implanted or treated/modified, e.g. in accordance with the simulation results (steps S7 to S23).
The first and second prior phases are common to the following steps of possible different methods for placing an implant inside a crystalline lens (ex: Figs 8A-N and 11A-M) or of a method for treating a crystalline lens without introducing any implant (ex: Figs 12A-F). Overall these prior phases may be used before any other method for putting in place an implant inside a crystalline lens or any other method for treating a crystalline lens without introducing any implant. The methods illustrated in Figs 8A-N, 11A-M and 12A-F and that are described after the performance of the first and second prior phases in the present description may alternatively be performed without using these first and second prior phases. Overall the diagnosis made on the patient's eye(s) makes it possible to give a clear picture of the aging grade of the crystalline lens(es). Based on such a diagnosis the most appropriate implant (intra cortical or intra capsular lens implant to replace in whole or in part the inner volume of the lens by an accommodative lens device) and/or treatment is selected. In addition, the diagnosis of the aging grade of a lens may identify other optical disorders than presbyopia. Thus, the above selection may also be made according to such other optical disorders.
According to the first step S1 (first phase), the patient is installed in a position where he/she is maintained immobile during the implementation of the method, e.g. he/she is seated or lies on an operating table.

Next step S2 (first phase) is a step of imaging the eye through conventional imaging method and associated equipment such as Optical Coherence Tomography (OCT).

By way of example, an apparatus called IOL Master 700 from Zeiss may be used for performing this imaging step. Such an apparatus provides a full-length OCT image showing anatomical details on a longitudinal cut through the entire eye.

Other OCT imaging methods and associated equipment as described in the following documents the content of which is hereby incorporated by reference: US6004314 ("Optical coherence tomography assisted surgical apparatus"), US6741359 ("Optical coherence tomography optical scanner"), US8908189 (" Systems and methods for swept-source optical coherence tomography") and US2016166147 ("Optical coherence tomography system") may also be used for performing imaging step S2.

Different conventional imaging methods and associated equipment may alternatively be used for performing imaging step S2.

In the course of imaging step S2 clinical or biometric data representative of the patient's eye geometry (physical characteristics of the eye) are obtained such as : nucleus posterior curvature radius, nucleus thickness, nucleus equatorial diameter, anteroposterior nucleus thickness ratio, nucleus anteroposterior position, pupil diameter, iris width (to scleral spur), eye length, vitreous size (height), intraocular pressure, anterior zonula insertion, anterior zonula attachment band width, equatorial zonula insertion, equatorial zonula attachment band width, posterior zonula insertion, posterior zonula attachment band width, ciliary scleral spur, ciliary apex anteroposterior position.

Other measurements/data may be derived from the above data through extrapolation such as scleral inner curvature radius, scleral external curvature radius, posterior scleral thickness and epithelium thickness.

The above makes it possible for the ophthalmologist (diagnosis) to identify and quantify the loss for visual accommodative amplitude of the eye, the residual accommodation capability, the axial displacement of the lens along the optical axis, as well as the ratio between the nucleus and the cortex and, more generally, the aging grade of the lens. Some of these results may alternatively be directly measured without the help of the ophthalmologist.

Next step S3 concerns the second phase and may be performed just after the first phase or subsequently, e.g. several days afterwards.

Step S3 is a step of simulation that is based on the results of the imaging and diagnosis which are representative of the patient's eye anatomy. Also statistical clinical data, for instance characterizing several physics-related parameters, may concern a sample of crystalline lens and may be used during this step. Such statistical data may be built on the basis of studies performed on representative samples of patients, accumulated imaging and classified in accordance with several criteria such as age, ethnicity, gender, etc.

For example, data characterizing a refractive index in relation to the age of a crystalline may be used

Simulation step S3 may be performed through optical simulation using a conventional simulator instrument such as the one described in US 2016296110 ("miniature simultaneous vision simulator instrument") the content of which is hereby incorporated by reference.

This step aims at simulating the vision through optical corrections set in the simulator instrument which are representative of the vision through a lens accommodative implant as though it were installed inside the patient's lens.

For the performance of the simulation the ophthalmologist selects the shape and dimensions of the implant to be put in place inside the patient's crystalline lens (curvature radii of the anterior and posterior parts, diameter of the equatorial plane, thickness along the polar axis etc. for both the most and the least accommodated positions of the lens, etc.), location of the implant in the crystalline lens (zone of the lens concerned, e.g. with respect to the different zones described with reference to Figures 7A-B) based on the above measurements/data, observations and diagnosis while possibly taking into account the description of the above embodiments (see for example Figs 3A-B, 4A-B, 5 and 7A-B).

Then test step S4 is performed. If the results of the simulation step are not in accordance with the aim to be achieved (increase in the lens flexibility and in the accommodative power of the aged patient's eye), then test step S4 leads to a new iteration and step S3 is repeated.

In a variant embodiment not illustrated here, step S3 may be achieved during the first phase.

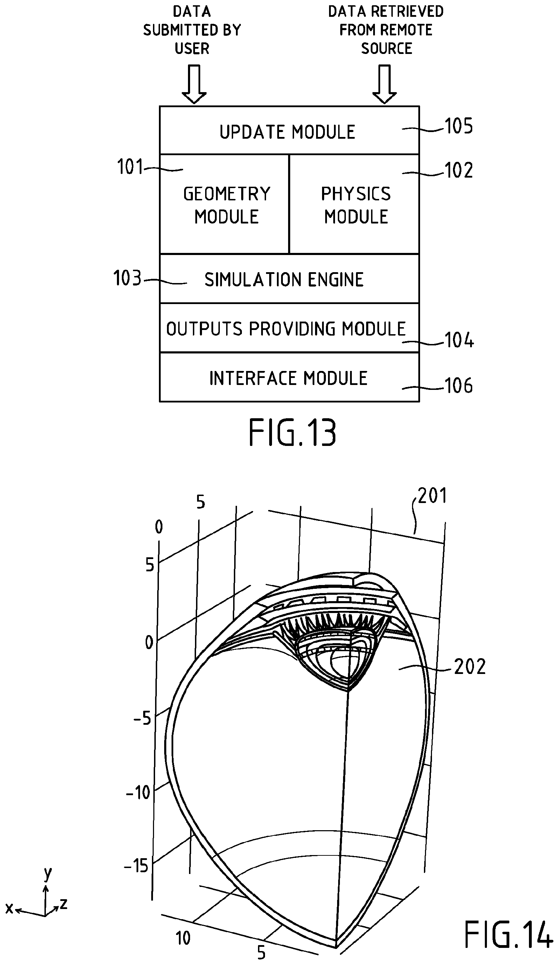

Steps S5 and S6 illustrate another alternative embodiment that may take place instead of step S3. According to this new embodiment the simulation may be achieved differently and combine clinical measurements performed on the patient's eye (eye imaging), possibly with statistical clinical data, with a generic mechanical and optical model of an eye for producing better quality simulation results. The generic mechanical and optical model of the eye that is used here will be more thoroughly described subsequently with reference to Figures 13 and following. This model integrates, on the one hand, a 3D representation of an eye with mechanical links between the eye's components and variables parameters that can be adjusted by a user or by automatic inputs from imaging and, on the other hand, a conventional calculation module which includes, in particular, physical equations, ray-tracing module etc. The conventional calculation module is for example a software product called Comsol Multiphysics.

Statistical clinical data as already described above in relation with step S3 is stored in a dedicated database that is part of the system 100 of Figure 13. Advantageously, the statistical data may be correlated to the patient to be treated by extracting from it mean values in relation to the patient's profile. Accordingly, an additional step of sorting the statistical data extracted from the database in relation to one or more characteristics that are specific to the patient to be treated, for instance the patient's age or the patient's gender, may be provided here before using such data.

Computational modeling step S5 includes an updating sub-step of the model and a calibration sub-step thereof.

In the updating sub-step measurements/data obtained in the course of imaging step S2 and possible statistical clinical data are entered into the generic mechanical and optical model as parameters of this model of the eye so that the model become faithfully representative of the patient's eye through computation. This updating sub-step of the model may concern parameters such as mechanical parameters and geometrical parameters (e.g. parameters relating to the relative positions between the elements/components of the model, parameters relating to the geometry of the elements/components etc.), parameters relating to optical and/or mechanical properties of materials/components etc...

Further a calibration sub-step of the model is performed through computation so that the optical properties of the latter be adapted to that of the patient's eye.

In particular, the refractive index of the lens is selected/adjusted through the model (by calculation) so that the latter correctly positions an image of an object on the retina in near and far vision so as to be representative of the same visual characteristics as those of the patient's eye.

To be noted that other parameters may be adjusted likewise during the calibration step.

After this calibration sub-step, the model is now representative of the patient's eye, in particular of the crystalline lens before surgery.

Next simulation step S6 may be performed. In this step, the ophthalmologist selects the features of a possible implant to be placed in the patient's lens (e.g.: shape, dimensions, location, material(s)/substance(s) used for the implant etc.) and enters them into the model in order to simulate the mechanical behavior of this implant in the eye's model, as though it were placed in the patient's eye. Practically, data generated by the calculation module of the model will inform the ophthalmologist of, e.g. possible concentration of stresses at a particular location in the lens, undesired deformation of the lens etc. If the results are not satisfactory, then at least one of the above features of the implant is modified and entered into the model and the above process is repeated as often as necessary.

Once the mechanical and geometrical aspects of the implant have been validated, optical results of the implant have to be checked.

Optical simulation is performed using the above calculation module and ray-tracing module which simulates the emission of optical beams, their passage through the simulated/modelled representation of the patient's eye with the simulated/modelled implant and the resulting beams after traversing the patient's implanted eye model ant their location/spot size on the retina.

According to the results of the test step S4, the optical simulation results may lead to validate the mechanically selected/defined implant or to alter the latter and perform again the previous steps S5 and S6, in particular the mechanical and optical simulation steps, until validating the features of an implant.

Once the simulation phase has been achieved the geometry and mechanical and optical properties of the implant that gave rise to the desired simulated optical results (restoration of flexibility and increased amplitude of accommodation) have been determined and the corresponding implant can then be selected on the shelves if available or planned for the manufacture if customized. In case no implant is to be inserted into the patient's lens, then the corresponding surgical method may be planned and performed (see for example the method of step S23 and Figures 12A-F).

Following steps S7 to S23 concern the third phase and possible surgical methods for treating/implanting the patient's crystalline lens in view of increasing visual accommodative amplitude in a patient's eye.

Two methods for placing an intra cortical lens implant in the patient's lens are described in steps S7 to S16 and illustrated in Figures 8A-N. Steps S7 to S10 are common to the two methods.

In step S7 an access or passage is created inside the lens (zone Z). More particularly, a channel 70 is formed in the cortex 22b (Fig 8B), e.g. by drilling, in order to provide access (e.g. syringe access) to the zone Z from outside the lens capsule. The thus formed cortical channel may exit outside the lens capsule or not depending on the method used for channeling and the aim to be achieved.

For instance, electromagnetic treatment/processing may be used for channeling and perforation of the lens capsule at one end of the channel may be performed at the same time.

Step S7 is used in the described two surgical methods but may be optional in relation with other surgical methods for treating the crystalline lens. For example, if the method does not use any implant (as in Figures 12A-F), then step S7 may be omitted.

More particularly, in step S7 the electromagnetic treatment may be a laser treatment. An example of an apparatus and associated equipment used for performing this step will be described subsequently with reference to Figure 10.

Figure 8B illustrates a laser beam B and a laser spot Ls that is focused on the lens. In Figure 8B, the channel 70 has been formed. However, at the very beginning of the laser process, the laser spot Ls is focused on a posterior region of the capsule (under the epithelium) and moves along a predetermined inclined direction so as to connect the outside of the capsule to the inner central zone of the lens which will be further treated/processed with a view to accommodating the intra cortical lens implant. The laser treatment/processing aims at removing the tissues and forming channel 70 connecting the zone Z to the outside surface of the lens capsule. More details on the laser treatment/processing will be given in connection with the following step.

Other alternative methods for lens channeling may be envisaged such as laser treatment using a laser apparatus as a cutting apparatus or instrument.

The posterior location of the lens channel makes it possible not to affect the epithelium. The side location enables easy access to the lens channel through the sclera.

Other alternative locations for the lens drilling/channeling may be envisaged.

It is to be noted that this step which is the first step of a surgical method may be carried out several hours or days after the last simulation step S3 or S6.

Figure 8C identifies in the lens 22 of Figure 8B a zone Z (partly illustrated in dotted lines) that is to be treated/processed before putting in place the intra cortical lens implant 40 in this zone. This zone Z here partially covers the nucleus and the cortex. The geometry and location of this zone are known from the previous steps, in particular S6, and are tailored to the patient.

Step S8 concerns the electromagnetic treatment of zone Z that uses, for example, the same apparatus and associated equipment as above.

In the present embodiment zone Z is laser processed so as to produce photoablation of the zone and create a cavity inside the lens. The operating laser parameters for photoablation will be described with reference to Figure 10. Photoablation is a conventional process used in eye's surgery. To be noted that step S7 illustrated in Figure 8B may also be performed by photoablation.

Figure 8C illustrates a laser spot Ls that is focused on a region of zone Z. The laser spot is programmed in the above apparatus to scan the whole zone Z, starting by the posterior part thereof (close to the posterior part of the cortex 22b) and moving forward towards the anterior part of the zone (close to the anterior part of the cortex) in the direction of the optical axis X. By analogy, the scanning process is carried out as in a known 3D printing process. The laser spot removes the tissues by transferring the laser energy to the zone Z where the tissues are vaporized. The zone Z is therefore laser processed by scanning successive layers, layer by layer as diagrammatically illustrated in Figure 8C (each layer is defined in a plane that is perpendicular to axis X). Operating parameters used for the photoablation will be provided subsequently.

In alternative embodiments zone Z may be processed differently for ablating the tissues of the zone, e.g. through radiofrequencies, UltraSounds, UV radiation, etc. For instance, a Phacoemulsification technique may alternatively be applied here.

Figure 8D represents the lens after core zone Z has been prepared for subsequent installation of the intra cortical lens implant.

Steps S7 and S8 may be inverted or performed at the same time.

In step S9 an incision I is made in the sclera 14 (one or more incisions may be made) so as to provide access to the lens from outside the eye. For instance a conventional surgical tool such as a calibrated knife K may be used as represented in Figure 8E.

To be noted that any cut, incision or opening made in the sclera, the cornea or lens should be as watertight as possible for subsequent quick sealing. For example a Z-shaped opening or openings in a sagittal view may be envisaged. Any cut, incision or opening may be made by any conventional technique.

This incision step may be omitted if no implant is put in place in the lens and only electromagnetic treatment/processing of the lens is performed to alter the latter so as to provide it with new accommodative amplitude (ex: Figs 12A-F). Steps S8 and S9 may be inverted in a variant embodiment.

Further step S10 is an extraction step of the natural material (tissues) of the lens that has been ablated in the zone Z as illustrated in Figure 8F. This step may include the perforation of the capsule if it has not been done during step S7.

Extraction of the material is performed through channel 70 connecting zone Z to outside surface of the lens capsule (once the channel has been opened to the outside either by photoablation or by any other surgical method/instrument) and through the sclera incision I using a conventional instrument that is connected to a phacoemulsification equipment. The phacoemulsification equipment is a microprocessor-based machine which controls fluid dynamics through the microprocessor. The equipment includes a peristaltic or Venturi type of pump for sucking out the lens material. Figure 8F partially represents such a pump with an aspiration chamber or reservoir 72 disposed outside the eye and connected to a flexible connecting duct 74 that has been previously inserted into the sclera incision I and the channel 70 so as to establish communication between the zone Z and the chamber 72. When the pump is operated the material and tissues debris are sucked out through the duct and reach the chamber where they may be collected or where they just go through before being collected or evacuated elsewhere as indicated by the arrow.

Such a technique is described for example in US 5154696 ("Phacoemulsification, irrigation and aspiration method and apparatus") and US2002099400 ("Method and apparatus for lenticular liquefaction and aspiration"), the content of which is hereby incorporated by reference.

After complete extraction a cavity 76 has been freed up inside the natural lens 22 so as to enable insertion of implant 40 therein. The shape of the outside outline of the intra cortical lens implant corresponds to the shape of the cavity (so that the implant is in contact with the cavity wall at any point) apart from the dimensions of the implant that may differ to some extent as will be further described.

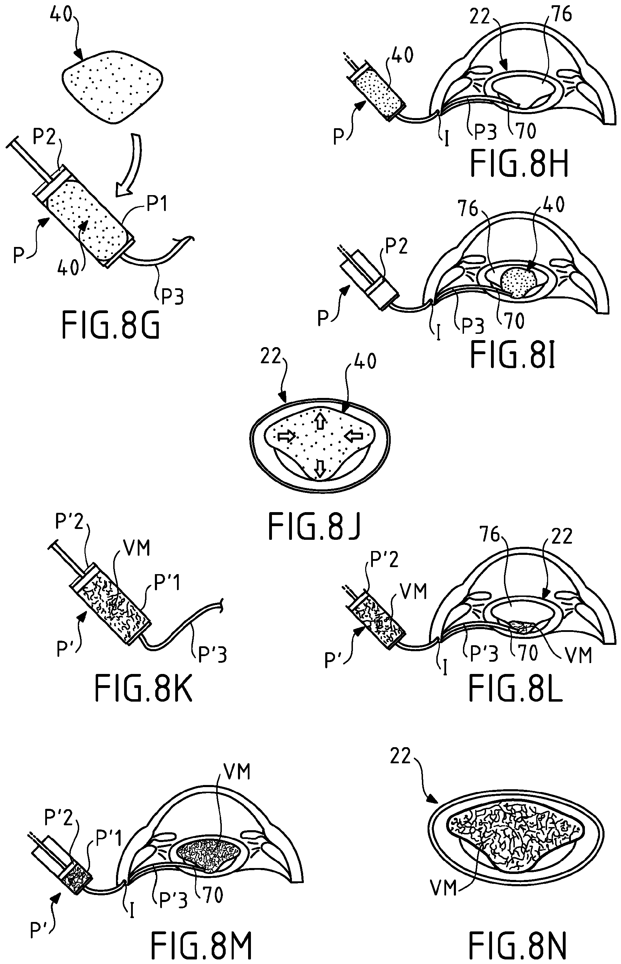

After these common steps S7-10, two surgical methods will now be described: a first method according to steps S11-13 (Figs 8G-J) and a second method according to steps S14-16 (Figs 8K-N).

According to the first method step S11, illustrated by Figure 8G, is a preparation step during which intra cortical lens implant 40 is introduced into a piston or syringe type device P. Device P comprises a chamber P1 and a piston P2 that is configured to axially slide within chamber P1 when pushed forward through its rod. A hollow insertion tip or cannula P3 with flexibility capabilities is connected to the device and in communication with the interior of the chamber. The implant 40 is introduced in the chamber before placing the piston. As the implant 40 is a flexible or malleable material or combination of materials with elastic or visco-elastic and cohesive properties it can be elastically deformed under external stress to be accommodated within the chamber and the piston is placed in the upper position. Alternatively, preparation step S11 may be omitted within the described method. In such a case, this step is performed beforehand: the device P is already pre-loaded with implant 40 and therefore ready for use by the surgeon as a kit assembly in the described method.

Next device P is connected to lens 22 by inserting tip P3 successively through sclera incision I and channel 70 during an installation step S12 (Figure 8H). Thus installed, the distal end of the tip opens out within cavity 76.

As illustrated by Figure 8I, the implant 40 is progressively inserted in the cavity 76 (step S13) by pushing forward the piston P2, which forces the implant to further elastically deform in order to be introduced inside tip P3, and pushed inside the latter along the whole length thereof until it enters into cavity 76. The implant 40 being pushed by the piston expands within cavity 76 as it progressively exits the distal end of tip P3 and the constraints exerted by the walls of the tip do no longer apply on the implant. The inserted intra cortical lens implant fills in the space within the cavity. This insertion or injection step (device P may be viewed as an injectable device in which the intra cortical lens implant or any other intra cortical lens implant according to the invention is loaded; a pre-loaded injectable device of this type may be envisaged) is made possible thanks to the elasticity or flexibility of the implant.

The shape of the implant 40 has greater dimensions than those of the internal cavity. Thus, when the intra cortical lens implant expands within the cavity it is maintained in a (slightly) compressed state and closely spouses the inner wall delineating the cavity. The difference between the volume of the intra cortical lens implant and that of the emptied cavity is for example between 2 and 5%.

A further aspect of the invention which does not depend on the shape and localization of the intra cortical lens implant within the lens is that a difference in volume between the implant and the clear cavity may induce a controlled deformation of the lens. The shape of the lens may then be changed/altered by this volume difference. The intra cortical lens implant may assume any convenient shape that makes it possible to induce a controlled desired deformation of the lens. The shapes of the intra cortical lens implant and the cavity lens will adapt to each other. A simulation process may be helpful to ensure that there will be mechanical contact at any point between the shape of the intra cortical lens implant and the lens cavity and that the induced deformation will be the desired one. In particular, the lens deformation may take place along the polar axis where the intra cortical lens implant is the most mechanically constrained, which induces a more curved lens as illustrated in Figure 8J by the internal arrows. In other words, the intra cortical lens implant causes the remaining natural tissues of the lens to be rounded up when inserted in the lens.

Where necessary, the cornea 12 may also be laser processed so as to modify the curvature of the cornea. In this case the laser apparatus is used as a cutting apparatus or instrument. This step (laser refractive surgery) may take place after the laser apparatus has been used at step S7 or S8 or at any other time. In the present embodiment, this step is achieved at the following step Sn (Control of Refraction and possible enhancement) in Figure 9. The operating parameters of the laser apparatus will be adapted to this specific step. When the crystalline lens has been modified by the above intra cortical lens implant, it has been given a new amplitude of accommodation with two new extreme shapes (one is more curved while the other is more flattened in the respective two extreme positions of the altered accommodative lens) and a new refractive index. It may happen that this new refractive index does not correspond to the desired one whatever the reason. In such a case recalibration of the eye has to be made so that the eye in a non-accommodated state (the lens is as flat as possible) may see far objects in an acceptable manner without any correction. Thus the whole eye optical system has improved since it has obtained an increase in accommodative amplitude for near vision. Then a change in the curvature of the cornea by refractive surgery turns out to be necessary to proceed to this recalibration. In contrast, if the new refractive index does correspond to the desired one, no change has to be made in the cornea. To be noted that step Sn may also be carried out after each of the other methods and, in particular, after steps S16, S20, S22 and S23. Further step Sn may provide data to the statistical clinical data to update the latter.

According to the second method of intra cortical lens implantation, step S14, illustrated by Figure 8K, is a preparation step during which one or more viscoelastic materials (a mixture or compound of viscoelastic materials), called hereinafter the viscoelastic material VM, (the viscoelastic material or materials have a refractive index that is suitable for being used in a crystalline lens) are introduced into a piston or syringe type device P'. The viscoelastic material VM has viscoelastic properties that confer flexibility to the core part of the lens as with the implant 40. Device P' comprises a chamber P'1 and a piston P'2 that is configured to axially slide within chamber P'1 when pushed forward through its rod. A hollow insertion tip or cannula P'3 with flexibility capabilities is connected to the device so as to be in communication with the interior of the chamber. The viscoelastic material VM (e.g. a gel) is introduced in the chamber before placing the piston. Alternatively, it may be preloaded in the device well in advance (for example, the viscoelastic material may be pre-loaded by the device manufacturer) and the preloaded device may then be ready for use by the surgeon.

Next device P' is connected to lens 22 by inserting tip P'3 successively through sclera incision I and channel 70 during an installation step S15 as in Figure 8L. Thus installed, the distal end of the tip opens out within cavity 76 (Figure 8L).

In the course of step S16 (Figure 8M) the fillable or injectable visco-elastic material VM is injected through tip P'3 to fill in the cavity 76 by pushing forward the piston P'2, which forces the flexible/malleable substance to elastically deform in order to be successively introduced inside tip P'3 and pushed inside the latter along the whole length thereof until it enters into cavity 76. The visco-elastic material being further pushed by the piston fills in the space within cavity 76 as it progressively exits the distal end of tip P'3 and the constraints exerted by the walls of the tip do no longer apply. Once the injection has been achieved the injected visco-elastic material VM perfectly spouses the shape of the inner wall delineating the cavity. The injected visco-elastic material VM behaves as an intra cortical lens implant, such as elastically deformable implant 40, and produces the same mechanical effects, in particular in terms of flexibility/softness so that it is able to provide new visual accommodative amplitude to the natural lens (this is an increase in the accommodative amplitude of the lens compared to the situation of the aged natural lens before the implantation).

Figure 8N illustrates the new implanted lens in which the visco-elastic material VM may have a shear modulus of, e.g. 500 Pa or 1000Pa (or other equivalent mechanical property). The shear modulus (or other equivalent mechanical property) for the visco-elastic material VM is generally less than that for an elastic implant as that of Figure 8J and, for example, less than 1500Pa. The surrounding part of the lens has for its part here a shear modulus between 1500-10000Pa. The intra cortical lens implant will not reach the value of 1500Pa if the surrounding part is known to be at 1500 Pa so as to respect the difference in the flexibility/rigidity between the intra cortical lens implant and the surrounding part; otherwise the spring effect would no longer apply.

Device P' may be viewed as an injectable device in which the visco-elastic material or any other substance according to the invention is loaded; a pre-loaded injectable device of this type may be envisaged.

For example, such a visco-elastic material may include siloxane (polysiloxane), hydrophilic gel.

Examples of substances called OVDs (acronym for "Ophtalmic, Viscoelastic Devices") that are suitable for the visco-elastic material VM can be found in the following documents, the content of which is hereby incorporated by reference:WO2016203381 ("viscoelastic preparation for use in surgical methods of ophthalmic surgery"), WO2006034383 ("viscoelastic solution or gel formulation, and methods of treating a body site with the same"), US5103840 ("viscoelastic collagen gel for ophthalmic surgery"), US4965253 ("viscoelastic material for ophthalmic surgery").

Examples of suitable OVDs may be provided by the company Zeiss under the following commercial names and the main technical characteristics (these substances are commercialized pre-packaged in a syringe and therefore ready to use):
- Visthesia 1.5% which is a viscoanesthetic OVD (substance: sodium hyaluronate; concentration: 1.5% NaHa, 1.0% lidocaine; volume: 0.8 ml inside the intracameral syringe; pH:7.0-7.6; osmolality in mOsmol/kg : 280-330 ; molecular weight in Da : 2,900,000 on average; pseudoplasticity index: 80; zero-shear viscosity in mPa.s : 187,000 on average);

- Visthesia 1.0 % which is a viscoanesthetic OVD (substance: sodium hyaluronate; concentration: 1.0% NaHa, 1.0% lidocaine; volume: 0.8 ml inside the intracameral syringe; pH:7.0-7.6; osmolality in mOsmol/kg : 280-330 ; molecular weight in Da : 2,900,000 on average; pseudoplasticity index: 58; zero-shear viscosity in mPa.s : 63,000 on average);
- Z-Hyalin plus which is a high-viscosity OVD (substance: sodium hyaluronate; concentration: 1.5% NaHa; volume: 1.0 ml inside the intracameral syringe; pH:7.2-7.6; osmolality in mOsmol/kg : 300-360 ; molecular weight in Da : 2,900,000 on average; pseudoplasticity index: 91; zero-shear viscosity in mPa.s : 250,000 on average);
- Z-Hyalin which is a high-viscosity OVD (substance: sodium hyaluronate; concentration: 1.0% NaHa; volume: 1.0 ml inside the intracameral syringe; pH:7.2-7.6; osmolality in mOsmol/kg : 300-350 ; molecular weight in Da : 2,900,000 on average; pseudoplasticity index: 50; zero-shear viscosity in mPa.s : 50,000 on average).

Also silicon oils may be employed for the visco-elastic material VM. Such substances are known today for their use in vitroretinal surgery.

Examples of suitable silicon oils are the following:
- ZEISS-RT SIL-OL 1000 commercialized by the company Zeiss (composition: 100% ultra pure polydimethylsiloxane; viscosity of 1,050 +/- 150 mPas at 25°C; specific weight of 0.97+/- 0.01 g/cm3 at 25°C; refractive index of 1.403 +/- 0.003 at 25°C; OH end group content: ≤ 100ppm; low molecular components (oligosiloxanes): ≤ 100ppm);
- SIL-1000-S Silicone Oil Syringe (a syringe of 10ml) commercialized by the company Dutch Ophtalmic, USA is an ultra-purified silicone oil which leads a maximum interfacial tension and minimizes interaction between tissues, cells and endo-tamponades media (viscosity: 1,000-1,500 mPas; refractive index: 1.40; specific gravity: 0.97 g/cm3 at 25°C; surface tension: 21 mN/m against air; interfacial tension: 40 mN/m against water);
- SIL-5000-S Silicone Oil Syringe (a syringe of 10ml) commercialized by the company Dutch Ophtalmic, USA is an ultra-purified silicone oil which leads a maximum interfacial tension and minimizes interaction between tissues, cells and endo-tamponades media (viscosity: 5,000 and 5,900 mPas; refractive index: 1.40; specific gravity: 0.97 g/cm3 at 25°C; surface tension: 21 mN/m against air; interfacial tension: 40 mN/m against water).

To be noted that fluids such as gas or liquids may be used alternatively according to the desired mechanical and optical results for filling in the cavity 76 of the lens and constituting the core part of the lens.

Figure 10 illustrates an embodiment of a system 80 used in the electromagnetic treatment/processing of a patient crystalline lens 22 according to the invention.

System 80 generates electromagnetic radiation and focuses it into the crystalline lens 22 of a patient eye.

System 80 comprises an apparatus 82 for generating electromagnetic radiation and a control device 84 such as a collimator for controlling and adjusting the direction of the generated radiation under the control of a data processing unit 86 or microprocessor and associated computer-readable storage media 88 (ex: memory or memories). Microprocessor unit 86 is also configured to operate apparatus 82 under the control of a computer program stored in computer-readable storage media 88.

In a particular embodiment, apparatus 82 is a laser, e.g. a femtosecond laser which delivers a beam 90 together with control device 84 focused onto crystalline lens 22 under the control of programmed microprocessor unit 86.

An example of femtosecond laser for performing laser phacofragmentation in the course of cataract surgery is commercially available from LenSx Lasers, Inc of Aliso Viejo, California and may be used for carrying out steps in the methods of Figure 9.

A Z8 Ziemer laser apparatus may alternatively be used. Such an apparatus is described in US2014098347 ("Ophtalmological device") the content of which is hereby incorporated by reference.

However, other types of laser may alternatively be used here.

A femtosecond laser has been used for ablating a lens during cataract surgery in US 2010/0191226. The content of this patent application is hereby incorporated by reference in particular as regards the laser ablation process.

Here, the femtosecond laser 82 is a laser of the YAG type and operates with the following parameters to carry out photoablation:
- wavelength between 800 and 2000 nm;
- frequency between 0.1 and 1 MHz;
- spot diameter between 1 and 10 µm;
- interaction time between 500 and 1000 femtosecond and
- energy between 0.1 and 100 µJ (the intensity may be deduced from the energy and interaction time values).

These parameters are also used in step S6 (lens opening) when the laser apparatus is employed.

In step S7 of Figure 9 and Figure 8C the femtosecond laser 82 proceeds from a posterior region in zone Z towards an anterior region thereof (postero-anterior direction) and from the periphery of zone Z to the center thereof.

The path or trajectory to be followed by beam 90 for ablating the natural material of zone Z is programmed by the surgeon according to the results of steps S2 to S5.

To be noted that another type of laser source may be used when a setting of optical offset has to be made in the cornea as described above. In this case an excimer laser may be used and operates in accordance with the following parameters:
- wavelength between 100 and 400 nm;
- frequency between 0.1 and 10 kHz;
- spot diameter between 100 and 1000 µm;
- interaction time between 0.1 and 100 nanosecond and
- energy between 0.1 and 10 mJ.

Such a type of excimer laser is described in US2016120700 ("Intastromal Corneal Reshaping Method and Apparatus for Correction of Refractive Errors Using Ultra Short and Ultra-Intensive Laser Pulses") the content of which is hereby incorporated by reference.

A laser apparatus commercialized by the Zeiss company under the commercial name VisuMax or MEL80 may be used here.

Another embodiment concerns an intra capsular lens implant that is intended to wholly fill in the capsular bag of a crystalline lens after the natural material (tissues etc.) contained in the latter has been wholly removed.

Figure 11A illustrates a crystalline lens 122 including the natural material 122a (different successive layers: nucleus, cortex and epithelium) contained within the capsular bag 122b. Natural material 122a is as described above with reference to Figure 2. The cornea 112, the sclera 114, the zonules 124 are represented as in Figure 8A.

Two methods for putting in place two new intra capsular lens implants respectively will now be described with reference to Figures 11B to 11H and steps S17 to S22 of Figure 9. Steps S17 to S19 are common to both methods.

In Figure 11B a surgeon creates one or more corneal incisions I' in the cornea 112 (step S17) to provide access to the anterior chamber 116 and lens 122 using a conventional instrument 126. Instrument 126 is here a calibrated knife. Alternatively, a laser apparatus may be used as a cutting instrument as already described above.

Next Figure 11C illustrates a step S18 of creation of an opening of the lens 122 by tearing and lifting a cap or cover of the lens capsule as indicated by 127. This opening is called capsulorexhis and is made using a conventional instrument 128 introduced through incision I'. Instrument 128 may be capsulorexhis forceps.

Alternatively, the instrument may be a femtosecond laser.

Figure 11D corresponds to a step S19 during which the whole content of capsular bag 122b is being fragmented e.g. in accordance with the phacoemulsification surgery using a probe such as an ultrasonic handpiece 130 with a titanium or steel needle. Ultrasonic handpiece 130 is introduced through incision I' and in contact with the lens material/tissues through opening O. The tip of the needle vibrates at ultrasonic frequencies in order to sculpt and emulsify the tissues while aspirating fragments through the tip thanks to a pump or aspirator equipment (a peristaltic or Venturi type of pump) as illustrated in Figure 11E. The conventional phacoemulsification equipment is a microprocessor-based machine which controls fluid dynamics through the microprocessor. Reference is also made to above step S10 of Figure 9 for completing this description.

Extraction of the fragments (Fig. 11E) is here performed using a conventional instrument that is connected to a phacoemulsification equipment.

Such a technique of phacoemulsification surgery is described for example in US 5154696 ("Phacoemulsification, irrigation and aspiration method and apparatus") and US2002099400 ("Method and apparatus for lenticular liquefaction and aspiration"), the content of which is hereby incorporated by reference.

In the end of the extraction step void has been created inside natural capsular bag 122b that remains intact but with an opening in its anterior part after capsulorhexis.

Figure 11F represents a new intra capsular lens implant 130 which comprises two parts/zones: a soft central or core part/zone 132 corresponding in this example to implant 40 of Figures 3 and 5 (other examples of implant may be envisaged provided they have the main characteristics as the above described implants regarding their softness/flexibility and their anterior and posterior portions) and a peripheral more rigid part/zone, called shell part, 134 which surrounds less rigid core part 132. The shell part 134 plays the role of the natural surrounding zone in Figure 5. The intra capsular lens implant may be considered as a one-piece unit even though the two parts have different mechanical characteristics (and possibly different materials). The intra capsular lens implant may be a bi-component implant with the two components being assembled together during the implant manufacture. The bi-component intra capsular lens implant may also be considered as a one-piece unit for the user.

In the present embodiment of this intra capsular lens implant, the materials used for each part/zone may be different (the materials for the two parts/zones are completely different in their composition): the material used for core part 132 may be the same as for implant 40 whereas the material used for the shell part 134 may be different. Alternatively, the same basic material may be used for both parts/zones but it is altered/ changed either by modifying its composition or by a subsequent treatment/process (such as heating the material for cross-linking/curing polymers differently depending on the parts/zones and therefore increasing the rigidity of the material concerned) with a view to obtaining different shear modulus.

Thus, the core part/zone may have a shear modulus less than 10 kPa, e.g. equal to 250 Pa, and the shell part/zone may have a shear modulus of 750 Pa.

By way of example, the materials used for the intra capsular lens implant may be, e.g. HEMA, silicon etc. More particularly, cross-linked polydimethyl siloxanes reinforced with silica may be used. Cross-linking (curing) may be obtained by different manners: radical, hydrosilylation, condensation etc.

Figure 11G illustrates a step S20 of insertion of intra capsular lens implant 130 inside the capsular bag 122b to fill in the void. Implant 130 is seized by a clamp instrument 136, introduced through corneal incision I' and placed within open capsular bag 122b so as to tension the inner wall of capsular bag 122b and come into close contact therewith. This insertion step is similar to that used in a process for inserting a conventional IOL in a lens where the haptics help to tension/stretch the capsular bag.

Implant 130 is therefore surrounded by the inner wall of capsular bag 122b apart from the upper region with opening O.

To be noted that in some instances, depending on the materials used for the intra capsular lens implant, conventional biological glue may be used to cause the capsule 122b to adhere to the implant 130.

Figure 11H represents the new crystalline lens with the intra capsular lens implant 130. The opening O is left unclosed. Thanks to this new intra capsular lens implant the lens is provided with increased accommodative amplitude as explained above for the other embodiments.

A method for putting in place another new intra capsular lens implant according to another embodiment of the invention will now be described with reference to Figures 11I-N and steps S21-22. This method takes over the general steps S17 to S19 of Figure 9 and Figures 11A-E as previously described.

Figure 11I illustrates both a piston or syringe-type device P" similar to that of Figure 8K and a closed flexible hollow envelope or shell C. Envelope C is represented in a cross-section in figure 11I and comprises a shell part or membrane Ca made in a soft material such as a material that is used for the envelopes of the breast prosthesis. For instance, silicon elastomer or an hydrophilic or hydrophobic polymer may be used for the material of the envelope. This material must be tear-resistant. Shell part Ca of Figure 11I has a variable thickness along its circumference so as to define the outline of a central cavity Cp. Shell part may have a substantially annular shape.

Envelope or shell C also comprises a core or central cavity Cp surrounded by this soft shell part Ca. In Figure 11I core cavity Cp does not contain any substance or material. Device P" is filled in with viscoelastic material such as in Figure 8K and differs from device P' in that flexible hollow tip P'3 is replaced by a rigid rectilinear hollow tip P'4.

In Figure 11J envelope C is handled by a clamp instrument 136 and introduced through incision I' so as to be placed within open capsular bag 122b and tension the latter so as to be in close contact with the inner wall of capsular bag 122b (step S21).

As represented in Figure 11K, sharp tip P'4 is introduced through incision I', perforates shell part Ca of envelope C until reaching the core cavity Cp so as to fill in the latter when piston P'2 is actuated as already described with reference to Figure 8M (step S22).

Once the core cavity Cp has been filled in with viscoelastic material, device P" is withdrawn.

The injected viscoelastic material may be the same as the above material VM, and is of the gel-type for example. The viscoelastic material may also be pre-loaded in an injection device.

Next an instrument 138 that is configured to suture/close the small aperture made in the shell part Ca by tip P'4 is installed inside the anterior chamber in close proximity to the shell part (Fig 11L). This instrument may generate, e.g. UV, thermal waves, RF etc. so as to cure/vulcanize the outer thin wall of the shell part.

In the present embodiment, this instrument may further cure/vulcanize the injected viscoelastic material VM so as to obtain the desired properties (viscosity etc.) for providing an intra capsular lens implant with relative flexibility/rigidity between the shell part Ca (more rigid) and the filled core part (more flexible). However, this further step may be omitted in some cases where the injected viscoelastic material VM has already the desired appropriate mechanical properties to form a soft core implant part.

Other instruments/methods may alternatively be used for suturing/closing the small aperture made in the shell as well as for curing/vulcanizing the injected substance and/or the substance in the annular portion. The degree of cure/vulcanization may vary according to the mechanical and optical results to be achieved.

Figure 11M illustrates the new crystalline lens with the intra capsular lens implant 140 which comprises shell part Ca surrounding core part Cb. The variable thickness of shell part defines in a complementary manner the outer shape of core part Cb. The two complementary shapes substantially reproduce the shapes of the implant 40 and surrounding natural tissues (mainly cortex) in the crystalline lens of Figure 5. The opening O is left unclosed. Thanks to this new intra capsular lens implant flexibility and therefore accommodative amplitude of the natural lens is increased as explained above for the other embodiments. The same mechanical properties as for intra capsular lens implant 130 are obtained here for intra capsular lens implant 140.

In a variant embodiment the shell part Ca of envelope C is already filled with a visco-elastic material such as a gel, e.g. of the silicone gel or silicone elastomer type. For example, an appropriate silicon elastomer may be polydimethylsiloxane, polydiphenyl-siloxane or some combination of the two. This variant embodiment does not modify the above-described following operations in connection with Figures 11J to 11M.

Optionally, the visco-elastic material in shell part Ca may also be cured/vulcanized to increase its rigidity (i.e. reduce its flexibility) relative to the filled core part during the step illustrated in Figure 11L.

In another variant embodiment, the core part Cb may be filled in by a fluid such as a liquid or a gas which will confer elasticity to the implant relative to a more rigid shell part.

In still another variant embodiment, the filled-in device P" with hollow tip P'4 is already assembled with envelope C (through a temporary fixation, e.g. of the mechanical type or by biological glue) and therefore ready for use as a kit assembly (pre-loaded device). Device P" with hollow tip P'4 can be easily separated from envelope C when the filling operation has been achieved, e.g. through a conventional instrument such as flat forceps.

In another embodiment the crystalline lens of the patient eye is treated/processed differently for restoring flexibility and increasing accommodative amplitude in a crystalline lens without placing any implant inside. The following surgical treatment/method may follow the steps S1 to S6 of Figure 9 and be represented by step S23. Optionally, step Sn may be carried out afterwards as already mentioned for other surgical methods.

Electromagnetic radiation is applied to the lens in order to create an inner shape or modify an inner zone of the lens, such as the shape of implant 40 or the zone it occupies in the lens (Fig 5) and bring to the lens appropriate properties in terms of softness/flexibility. The remainder of the lens remains unmodified as in the Figures 3 and 5 embodiment.

Figures 12A and 12B illustrate lens 22 of Figure 2 in a sagittal view and front view respectively.

Electromagnetic radiation is applied to the specific zone of the lens identified by zone Z that is identical to that of Figure 8C in order to modify the mechanical characteristics of this zone by breaking the links at the macromolecular level and the links between the layers so as to increase slip between the layers. In other words the structural resistance of this zone to the deformation is reduced. Such a treatment also modifies the optical properties (e. g. by shifting the refractive index) of the treated zone.

To be noted that the present description of this embodiment applies to any shape/zone internal to the crystalline lens and that provides the above-described technical effects and functions of the implant according to the invention (e.g. the mechanical implants, their shapes and locations within the crystalline lens as well as the mechanical and optical properties as illustrated in Figures 3A-B, 4A-B and 5). In particular, the present description also applies to the volumes and zones described in relation with Figures 7A-B.

Figures 12C and 12D illustrate an example of electromagnetic treatment of zone Z on the views of Figures 12A and 12B respectively in accordance with step S23 of Figure 9.

An example of electromagnetic radiation treatment system is given by the use of a femtosecond laser as that described above in relation with the ablation process and Figures 8B-C and 10.

As illustrated by Figures 12C and 12D the laser beam produces a laser beam B with a laser spot Ls which is first focused on the posterior part of zone Z. As explained above in relation with Figures 8B and 8C, the laser spot follows a predetermined trajectory or scan path from the posterior part of zone Z towards the anterior part thereof so as to homogeneously process the whole zone by successive smaller zones. Here, the laser spot describes a spiral path and as represented a first region R has already been laser processed at the posterior part of zone Z. S" is a partial enlarged view of an example laser pattern applied to the zone. The description of Figures 8B and 8C may also apply here as regards details of the operation. The operating parameters are however different. The coordinates of the spot are controlled by the processor of the apparatus in accordance with the predetermined scanning pattern.

The result of the laser treatment/processing is illustrated in Figure 12E (front view) and Figure 12F (sagittal view) where the core 150 has been achieved. Core 150 has the same or similar features as above implant 40. In particular, it has softer/more flexible tissues and a higher refractive index than the natural surrounding part 22'b of the new lens 22'. Such a method for altering/modifying lens 22 is not invasive.

In order to modify the crystalline lens according to the present embodiment the femtosecond laser 82 of Figure 10 has the following characteristics and operating parameters:
- wavelength between 800 nm and 2000 nm;
- frequency between 1 Hz ad 10 MHz ;
- spot diameter between 0.5 µm and 5 µm;
- interaction time between 100 and 500 femtosecond;
- energy between 0.1 and 10 nJ.

The advantage in this embodiment is that no invasive action has to be performed on the lens to correct presbyopia and /or any other optical disorder or disease.

The present invention (either as an implant or through a non-invasive method for altering locally the flexibility and possibly the refractive index of the lens as in Figs 12A-F) makes it possible to increase the visual accommodative amplitude of an aged natural lens by more than 1 diopter and for instance until 3 or 5 diopters. On average, an increase lying between 1.5 and 4.5 may be envisaged.

The above-mentioned model used in connection with simulation step S6 of Figure 9 will now be described with reference to Figures 13 to 17.

A computer system for simulating visual accommodation (hereinafter referred as "the system") comprises at least one processor and at least one computer-readable storage media. In an embodiment, the system includes a microcomputer whereas, in another embodiment, it includes a professional workstation, a server, a mainframe, a supercomputer or a combination of such systems. The computer-readable storage media of the system, for instance a memory, is encoded with instructions that, when executed by the processor, enable a simulation system for simulating visual accommodation, whose parameters, models, computations, results and advantages are described below.

As shown on figure 13, the system 100 comprises five main components, namely a geometry module 101, a physics module 102, a simulation engine 103, an output providing module 104, an update module 105 and an interface module 106.

The geometry module 101 allows to set a three-dimensional geometrical model of an eye. The three-dimensional geometrical model delineates volumetric boundaries of several physiological entities of an eye. The physiological entities whose volumetric boundaries are thus distinctively defined by the geometrical model are, preferably, the crystalline lens, the zonules, the ciliary muscle, the sclera, the cornea, the vitreous body and the aqueous humor. Accordingly, the geometrical model defined by the geometry module 101 results from an assemblage of several distinct geometries, each one relating to a specific physiological entity of an eye.

All physiological entities involved in the process of visual accommodation are defined by the geometrical model specified by the geometry module 101 of the system 100. As such, the geometry module 101 allows to faithfully transpose the geometry of a real eye and, therefore, it contributes to greater accuracy of the simulation results of visual accommodation which are provided by the system 100.

The crystalline lens as defined by the geometrical model is divided in four subparts, which are the nucleus, the lens cortex, the lens capsule and the lens epithelium. From a geometrical point of view, each subpart is defined as a solid generated by cross sectional continuous curves, forming a bi-convex aspheric and not necessarily regular domain. Each curve is the representation of a 5^{th} order polynomial I, where I is the considered plan of the cross section. The crystalline lens is thus defined as a set of four of such volumes nested together. The geometrical model further defines an offset of distance between each subpart along the crystalline lens polar axis

With respect to the sclera and the cornea, the geometrical model defines the sclera's shape and the cornea's shape as a volume generated by an ensemble of 5^{th} order polynomial curves.

With respect to the zonules, since the zonular fibers form a complex web sustaining the lens, connecting it with the ciliary muscle, and linking the ciliary muscle to the sclera, the geometrical model defines three zonula groups (posterior, equatorial, anterior). Each group is made of two zonula rings delimiting the insertion thickness. The geometrical model further defines several intersections where each of these groups intersects with the crystalline lens and/or the ciliary muscle.

With respect to the ciliary muscle, which is a structure of multiple fibers orientation, the geometrical model defines its specific shape as bounded by a skin, which is defined through a generated central part of revolution from which are derived multiple discrete segments of extrusion.

The geometrical model further defines at least one area where the ciliary muscle is fixed to the sclera, points or areas at which it interacts with the zonules and further geometrical aspects designed to allow the ciliary muscle to slide on the choroid during the accommodation process.

The geometry module 101 interacts with the interface module 106 to generate a window of the graphical user interface provided by the interface module 106, in which a three-dimensional representation of the geometrical model is displayed.

The graphical user interface generated by the interface module 106 provides functionalities enabling data submission, including numerical and graphical inputs. Accordingly, the geometrical model managed by the geometry module 101 may be modified by means of graphical inputs performed directly on the three-dimensional representation displayed in the graphical user interface, thereby allowing a sort of "WYSIWYG - what you see is what you get" type of interaction. This feature is particularly advantageous in situations where, for instance, the geometrical model must be aligned to in-vivo measurements of real eyes' geometries. In that sense, the system 100 provides an improvement in terms of man-to-machine interaction, thereby enhancing its efficiency when used, for instance, in research & development or therapeutic environments.

Figure 14 schematically depicts at least a part of a window 201 of the graphical user interface provided by the interface module 106, in which a partial three-dimensional representation 202 of the geometrical model is displayed.

The role of the physics module 102 is to define a physics-related environment in relation to the geometrical model managed by the geometry module 101. As such, it allows the simulation results provided by the system 100 to rely on a combination of geometry and physics, by means of a precise definition of mechanical and optical properties in relation to given eyes' geometries. To this end, the physics module 102 allows setting of numerous physics-related parameters to characterize optical and mechanical properties of, preferably, all parts defined by the geometrical model.

Advantageously, the physics module 102 processes data to assign mechanical and/or optical properties to specific points, areas or volumes defined by the geometrical model. Thus, the physics module 102 provides fine tuning capabilities for setting the physics-related environment, thereby contributing to the provision by the system of more accurate simulation results.

With respect to the mechanical properties of the crystalline lens, the physics module 102 allows to set a physics-related parameter that characterizes its stiffness. Preferably, the physics module 102 allows to set distinct physics-related parameters, some or all relating to stiffness, with respect to specific areas of the nucleus, the lens cortex, the lens capsule or the lens epithelium. Advantageously, a physics-related parameter may be set only for specific points, areas or volumes of those subparts.

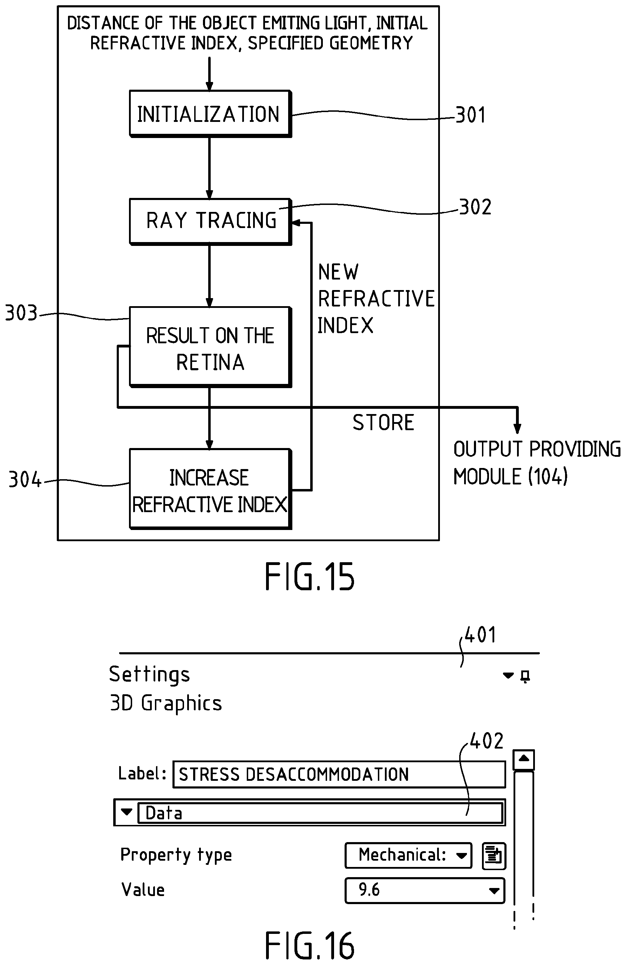

With respect to the optical properties of the crystalline lens, the physic module 102 allows a physics-related parameter of refractive index to be set. Preferably, setting of the refractive index parameter involves determination of the refractive index parameter based on a computation of the sum of cumulated light received by the retina, for different concentric areas characterizing the sharpness of focus, when considering, for a given geometry, a situation where light is emitted by a point located one meter away from the cornea. This computation that may be performed by the physics module 102 in order to determine the refractive index parameter is depicted by figure 15.

In a step 301, the physics module 102 retrieves the geometrical model defined by the geometry module 101, a value characterizing the distance of the object emitting light and assigns an initial value to the refractive index parameter. In a step 302, the physics module 102 generates a ray tracing and, in a step 303, the physics module 102 determines a corresponding result on the retina by computing the cumulated light received by the retina. In a step 304, the physics module 102 increases the refractive index parameter by a pre-defined offset and repeats the steps 302 and 303 until a defined stop value of refractive index. Once it has performed those steps for several different values of refractive index, the physics module 102 sets the refractive index parameter by selecting the value which produces the sharpest result on the retina.

With respect to the mechanical properties of the sclera, the physics module 102 allows to set a physics-related parameter to characterize its stiffness, preferably by means of a linear material model.

With respect to mechanical properties of the zonules, the physics module 102 allows to set a physics-related parameter that characterizes the stiffness of its anterior, posterior and equatorial parts. Preferably, the physics module 102 allows setting of a physics-related parameter that characterizes an elongation level of the posterior part of the zonules.

With respect to mechanical properties of the ciliary muscle, the physics module 102 allows to set a physics-related parameter that characterizes its core's elasticity and its skin's rigidity. Moreover, the physics module 102 allows to set a physics-related parameter that characterizes weaknesses at some specific locations of the muscle's skin, thereby allowing to characterize the behavior of the muscle's skin which, during accommodation, changes its shape when submitted to the traction of the posterior part of the crystalline lens. Preferably, the physics module 102 also allows setting of a physics-related parameter that characterizes contraction and/or elongation level with respect to the whole ciliary muscle and/or with respect to only a part of it. Accordingly, the visual accommodation process may be simulated by means of modifications applied to the physics-related parameters characterizing stiffness of at least some parts of the ciliary muscle.

With respect to mechanical properties of the aqueous humor, the physics module 102 allows setting of a physics-related parameter that characterizes its dynamic viscosity, density or elasticity. Moreover, the physics module 102 allows to set several physics-related parameters that characterize fluidic properties of anterior and posterior chambers, inflow from the ciliary body at a constant mass flow rate and an outflow rate.

With respect to optical properties of the aqueous humor, the physics module 102 allows a physics-related parameter that characterizes its refractive index to be set.

With respect to mechanical properties of the vitreous body, the physics module 102 allows a physics-related parameter that characterizes its elasticity and/or an index of refraction to be set. Preferably, viscoelastic properties of the vitreous body are characterized by the physics-related module 102 via a physics-related parameter which is based on two Kelvin-Voigt model in chain with a damper.

With respect to optical properties of the vitreous body, the physics module 102 allows a physics-related parameter that characterizes its refractive index to be set.

In terms of man-to-machine interaction, the physics module 102 interacts with the interface module 106 to generate, as shown by figure 16, a window 401 of the graphical user interface provided by interface module 106. The window 401 includes at least input means 402 that may be operated to adjust all the physics-related parameters defined by the physics module 102. Alternatively, or cumulatively, the window 401 includes input areas to facilitate numerical inputs, thereby allowing also a "WYSIWYG" type of man-to-machine interaction. As mentioned above, the graphical user interface provides functionalities for management of any type of man-to-machine interactions. This applies to the window 401, which may thus allow graphical inputs to be submitted by means of pointing devices and numerical inputs to be specified by means of dedicated input areas.

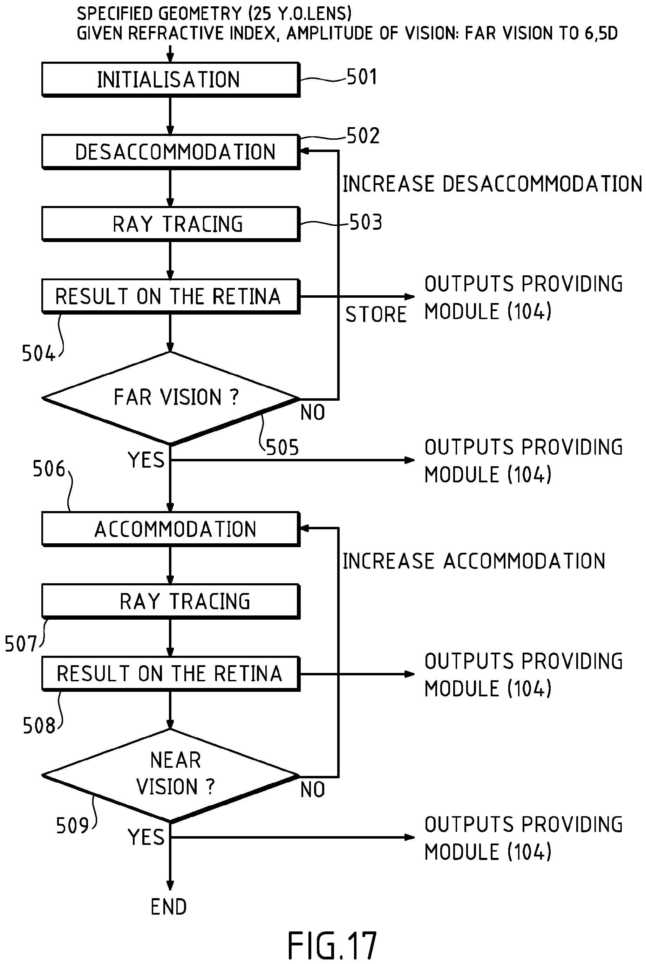

The role of the simulation engine 103 is to combine the geometrical model defined by the geometry module 101 with the physics-related environment defined by the physics module 102. As such, the simulation engine 103 generates a simulation model which, to produce graphical or numerical results with respect to a process a visual accommodation, duly considers geometrical and physics-related aspects. An example of a process of combining geometrical and physics-related aspects that may be performed by the simulation engine 103 is depicted by figure 17

In a first step 501, the simulation engine 103 retrieves from the geometry module 101 a geometrical model characterizing a young emmetropic eye, preferably a twenty-five years old eye. In parallel, the simulation engine 103 retrieves from the physics module 102 the refractive index parameter of the crystalline lens corresponding to the given geometry.

In a step 502, the simulation engine 103 operates the geometrical model to characterize a tension on the posterior part of the zonules, which induces a deformation of the crystalline lens and, with respect to the given geometry of a twenty-five years old eye, the simulation engine 103 determines the force needed to achieve far vision. Accordingly, during this step 502, the simulation engine 103 sets an initial value of tension applied on a posterior part of the zonules to achieve far vision. In a step 503, the simulation engine 103 generates a ray tracing and, in a step 504, it determines a corresponding result on the retina by computing the cumulated light received by the retina. In a step 505, the simulation engine 103 determines if far vision is achieved or not. If it is not the case, in order to characterize greater tension applied on the posterior part of the zonules, the simulation engine 103 increases the value of tension applied on the posterior part of the zonules using a pre-defined offset and steps 502-505 are repeated until the simulation engine 103 determines in step 505 that far vision is achieved. When the simulation engine 103 determines that far vision is achieved, it transmits the value of tension applied on the posterior part of the zonules to achieve far vision to the outputs providing module 104.

Then, in a step 506, considering the far vision achieved, the previous determined value of tension applied on posterior part of the zonules to achieve far vision remain identical, and the simulation engine 102 applies a first initial value of contraction of the ciliary muscle. In a step 507, the simulation engine 103 generates another ray tracing for a given distance of ray-emission corresponding to a close object and, in a step 508, it determines the result on the retina. Then, in a step 509, the simulation engine determines if near vision is achieved or not. If it is not the case, in order to characterize greater contraction value of the ciliary muscle, the simulation engine 103 increases the value of contraction applied on the ciliary muscle using a pre-defined offset and steps 506-509 are repeated until the simulation engine 103 determines in step 509 that near vision is achieved. When the simulation engine 103 determines that near vision is achieved, it transmits the value of contraction applied on the ciliary muscle to achieve near Vision to the outputs providing module 104.

By means of this process, the simulation engine 103 is thus able to determine the parameters of the tension and contraction characterizing an accommodation amplitude of a young eye. Moreover, considering the hypothesis that the efforts for visual accommodation remain similar for young and old people, the simulation engine 103 may for instance evaluate the accommodation amplitude of an old eye by applying the previously determined parameters of tension and contraction to the geometrical model and the physics related environment of an older eye.

The role of the output providing module 104 is to retrieve results data that are generated by the simulation engine 103 and to interact with the interface module 106 to present these results as numerical and graphical outputs.

The simulation results generated by the simulation engine 103 and retrieved by the outputs providing module 104 include, preferably with respect to all physiological entities defined by the geometrical model, data that relates to forces, mechanical stresses, deformations or displacements. With respect to optical domains, the results include data that relate to optical changes and, in relation to visual accommodation, the results retrieved by the outputs providing module 104 include at least data defining a value of accommodative amplitude.

The outputs providing module 104 retrieves data from the simulation engine 103 and processes the data to feed the interface module 106. In this respect, the interface module 106 may present the results via one or more windows. Preferably, results are presented in a graphical or numerical form, the choice of format being made in accordance with a set of pre-defined formats which specifies, for each result that may be provided by the simulation engine 103, an appropriate format to be used.

A role of the update module 105 is to monitor user inputs and, if necessary, to update the geometrical model or to the physics-related environment accordingly. In this respect, the update module 105 continuously interacts with the interface module 106 so that, when the former is alerted by the latter that user inputs are submitted, it determines whether such inputs represent changes that apply to the geometrical model, so-called geometry-related changes, or to the physics-related environment, so-called physics-related changes. For instance, when the interface module 106 detects that user inputs are submitted via the three-dimensional representation 202 of the geometrical model displayed in window 201, it alerts the update module 105 that, in turn, modifies the geometrical model defined by the geometry module 101. In other words, numerical and graphical inputs are continuously monitored by the interface module 106 and processed in real-time by the update module 105 to update the simulation environment (geometry and/or physics). Thus, the update module 105, by allowing changes made to the simulation environment to be continuously monitored and processed by the system, provides automatic updating mechanisms which contribute to increase efficiency of the system.

Another role of the update module 105 is to retrieve data from remote data sources, for instance medical measurement devices or a medical imaging devices. As previously stated, the system 100 may indeed be part of a computerized therapeutic environment, in which it may be connected to medical devices from which, by means of functionalities provided by the update module 105, it may retrieve geometrical or physics-related data.

In such situations, the update module 105 is configured to interact with interfaces of those devices and it updates the geometrical model defined by the geometry module 101 or the physics-related environment defined by the physics module 104 in accordance with data that it retrieves from those data sources. Thus, by allowing for instance in-vivo data to be directly retrieved and processed by the system, the update module 105 also contributes to increase efficiency of the system 100, especially when it is used in such a computerized R&D or medical environment.

In a general manner, update module 105 is used in the computational modelling step S5 of Figure 9 together with geometry module 101 and physics module 102 (mechanical and optical) to update the generic model and make it representative of the patient's eye. More particularly, update module 105 is configured to update the geometrical model hosted by the geometry module 101 and the physics-related environment stored by physics module 102 in accordance with data entered into the system.

The physics module 102 makes it possible to perform the calibration sub-step of step S5 of Figure 9 and in particular to determine the refractive index as illustrated in Figure 15 and described above. Also visual accommodation may be computed as illustrated in Figure 17 and described above.

Update module 105 permanently controls any new input information/data in connection with a lens implant or, more generally, any eye surgical method. New input information/data may be provided by the surgeon through interface module 106. Update module 105 cooperates with the simulation engine 103 to simulate the mechanical and optical behavior of an implant in the model's patient eye (step S6 of Figure 9).

In particular, simulation engine 103 may perform the computational steps of Figure 17 based on the above new information/data and provides numerical and/or graphical outputs through outputs providing module 104. These outputs are then analyzed, e.g. by the surgeon, to determine if the lens implant or, more generally, any eye surgical method provides the desired results in terms of flexibility, visual accommodation and any other optical correction.

In contrast to most system for simulating visual accommodation, which often focus only on the role of the crystalline the lens, the system 100 addresses with a great level of details numerous physiological entities that are present in a real eye. Moreover, as explained above, the geometry of the eye may be controlled from measured data or whatever data that is entered, which allows the system 100 to be able to grasp most of the possible geometrical combinations that may be found in real eyes. The system 100 is thus more efficient when used, for instance, in a R&D or therapeutic environment because, in such a situation, simulation results are useful only if they are able to reflect in an accurate manner the physiological reality.

## Claims

1. An intra cortical lens implant having a longitudinal axis corresponding to a polar axis of the intra cortical lens implant, wherein the intra cortical lens implant comprises an anterior part and a posterior part that extend axially along the polar axis, the anterior part having an anterior pole (A) located on the longitudinal axis and the posterior part having a posterior pole (E) located on the polar axis, the anterior part and the posterior part extending each radially relative to the polar axis on either side thereof, the anterior part and the posterior part having each two portions located on both sides of the polar axis respectively when viewed in a plane including the polar axis, called sagittal plane, each portion of the anterior part having a radial extension that increases from the anterior pole (A) to a point (B, B') where the anterior part ends and the posterior part begins, each portion of the posterior part having a radial extension that decreases from the point (B, B') where the posterior part begins to the posterior pole (E), the outer outline of each portion of the anterior part forming a curve having a radius of curvature that is greater at the anterior pole (A) than at the point (B, B'), the intra cortical lens implant being made of one or more materials that have elastic or visco-elastic and cohesive properties in a solid state such that the shear modulus is greater than 10 Pa and less than 10 kPa, the one or more materials having a refractive index that is suitable for being used in a crystalline lens.

2. The intra cortical lens implant of claim 1, wherein the anterior part has a convex shape when viewed in a sagittal plane.

3. The intra cortical lens implant of claim 1 or 2, wherein the outer outline of each portion of the anterior part is a continuous curve with a radius of curvature R that overall decreases from the anterior pole (A) to point (B, B') in a sagittal plane.

4. The intra cortical lens implant of any of claims 1 to 3, wherein each portion of the posterior part has an outer outline that forms a continuous curve between the point (B, B') and the posterior pole (E) when viewed in a sagittal plane.

5. The intra cortical lens implant of claim 4, wherein the outer outline of each portion of the posterior part includes two points (C, D), respectively (C', D'), located on the curve between the point (B), respectively (B'), and the posterior pole (E) and that form two inflexion points for the curve.

6. The intra cortical lens implant of claim 5, wherein the two points (C, D), respectively (C', D'), are located on a straight line between the point (B), respectively (B'), and the posterior pole (E).

7. The intra cortical lens implant of claim 5 or 6, wherein each curve between the point (B), respectively (B'), and the posterior pole (E) includes two side portions (B, C), respectively (B', C'), and (D, E), respectively (D', E), flanking a central portion (C, D), respectively (C', D'), the two side portions having a curvature of the same type, convex or concave, and the central portion having a curvature of the opposite type, concave or convex.

8. The intra cortical lens implant of any of claims 1 to 7, wherein the intra cortical lens implant has an axi-symmetrical shape relative to its polar axis when viewed in a sagittal plane.

9. The intra cortical lens implant of any of claims 1 to 7, wherein the intra cortical lens implant has not an axi-symmetrical shape relative to its polar axis when viewed in a sagittal plane.

10. The intra cortical lens implant of any of claims 1 to 9, wherein the intra cortical lens implant has an axi-symmetrical shape relative to its polar axis when viewed in a front plane that is perpendicular to the sagittal plane.

11. The intra cortical lens implant of any of claims 1 to 10, wherein the intra cortical lens implant has not an axi-symmetrical shape relative to its polar axis when viewed in a front plane that is perpendicular to the sagittal plane.

12. An intra capsular lens implant intended to wholly fill in the capsular bag in an eye, wherein the intra capsular lens implant comprises a core part and a shell part that surrounds the latter, the intra capsular lens implant having a longitudinal axis corresponding to a polar axis of the intra capsular lens implant, wherein the core part comprises an anterior part and a posterior part that extend axially along the polar axis, the anterior part having an anterior pole (A) located on the polar axis and the posterior part having a posterior pole (E) located on the polar axis, the anterior part and the posterior part extending each radially relative to the polar axis on either side thereof, the anterior part and the posterior part having each two portions located on both sides of the polar axis respectively when viewed in a plane including the polar axis, called sagittal plane, each portion of the anterior part having a radial extension that increases from the anterior pole (A) to a point (B, B') where the anterior part ends and the posterior part begins, each portion of the posterior part having a radial extension that decreases from the point (B, B') where the posterior part begins to the posterior pole (E), the outer outline of each portion of the anterior part forming a curve having a radius of curvature that is greater at the anterior pole (A) than at the point (B, B'), both the core part and the shell part being made of one or more materials that have elastic or visco-elastic and cohesive properties in a solid state such that the shear modulus is greater than 10 Pa and less than 10 kPa, or the shell part being made of one or more of the previous materials and the core part being made of a fluid, both the one or more materials and the fluid having a refractive index that is suitable for being used in a crystalline lens.

13. The intra capsular lens implant of claim 12, wherein the shell part is more rigid than the core part.

14. The intra capsular lens implant of claim 13, wherein the shell part is made of a material of which the shear modulus is greater than that of the core part.
